(19) Europäisches Patentamt
European Patent Office
Office européen des brevets

(11) Publication number: **0 520 674 B1**

# (12) EUROPEAN PATENT SPECIFICATION

(45) Date of publication of patent specification: **16.08.95**

(51) Int. Cl.[6]: **C07D 319/20**, C07D 407/12, C07D 493/04, C07D 405/12, A61K 31/335, //(C07D493/04, 319:00,311:00)

(21) Application number: **92305535.4**

(22) Date of filing: **17.06.92**

(54) **Antipsychotic benzodioxan derivatives.**

(30) Priority: **21.06.91 US 719882**
**21.06.91 US 719887**

(43) Date of publication of application:
**30.12.92 Bulletin 92/53**

(45) Publication of the grant of the patent:
**16.08.95 Bulletin 95/33**

(84) Designated Contracting States:
**AT BE CH DE DK ES FR GB GR IT LI LU NL PT SE**

(56) References cited:
**EP-A- 0 175 541**
**GB-A- 1 096 301**

**Algemeine und spezielle Pharmakologie und Toxikologie, S. Auflage, Bibliographisches Institut, Mannheim 1987, pages 519-521, und 548-552.**

(73) Proprietor: **AMERICAN HOME PRODUCTS CORPORATION**
**Five Giralda Farms**
**Madison, New Jersey 07940-0874 (US)**

(72) Inventor: **Stack, Gary Paul**
**525 Brookfield Lane**
**Ambler,**
**Pennsylvania 19002 (US)**
Inventor: **Abou-Gharbia, Magid Abdel-Megid**
**6 James Hayward Road**
**Glen Mills,**
**Pennsylvania 19342 (US)**
Inventor: **Andree, Terrance Harold**
**2623 Red Gate Drive**
**Doylestown,**
**Pennsylvania 18901 (US)**
Inventor: **Scherer, Noreen Theresa**
**3215 Unruh Street**
**Philadelphia,**
**Pennsylvania 19149 (US)**

**EP 0 520 674 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Connelly, Michael John**
**c/o Patent Department**
**Wyeth Laboratories**
**Huntercombe Lane South**
**Taplow**
**Maidenhead**
**Berkshire, SL6 0PH (GB)**

## Description

## Background of the Invention

Indian J. Chem., Sect B 1982, 21B(10), 914-18 discloses a series of (aminophenoxy)(arylpiperazinyl)-propanes (I) with potent CNS depressant, hypotensive, α-adrenoceptor blocking, antiinflammatory, and diuretic activities. The compound in which RNH = p-$NH_2$ and $R^1$ = o-MeO is a potent neuroleptic.

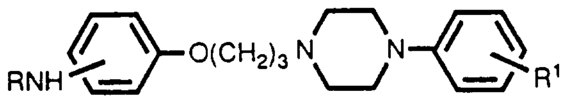

(I)

Jpn. Kokai Tokkyo Koho JP 57,142,972 and Fr. Demande FR 2,477,542 claim compounds of formula (II) as antihistaminic, anti-aggressive, and adrenaline antagonistic agents, useful as central nervous system agents. R is H, alkyl, phenylalkyl, alkenyl, alkynyl; Z is N-phenylimino, (un)substituted benzylidene; n is 0 or 1; $Z^1$ is alkylene; and $Z^2$ is CO, CH(OH), (un)substituted vinylene or ethylene.

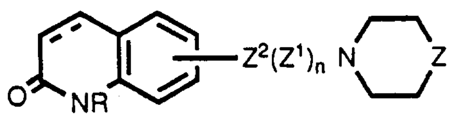

(II)

European Patent Application EP 170,213 discloses a series of glutarimide derivatives of benzodioxan methanamine as antianxiety and antihypertensive agents. Fozard et. al. Br. J. Pharmacol. 90, 273P (1987) disclose 8-[4-(1,4-benzodioxan-2-ylmethylamino)butyl]-8-azaspiro[4.5]decane-7,9-dione (MDL 72832) as a selective and stereospecific [(-)-MDL 72832 binds 32 times as much as the dextrorotary isomer at the 5-$HT_{1A}$ receptor site] ligand for 5-$HT_{1A}$ receptors.

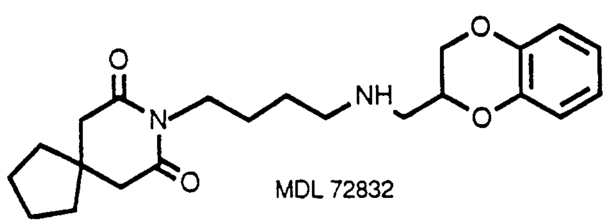

MDL 72832

European Patent EP 236,930 discloses a series of 2-substituted-alkyl-1,2-benzisothiazole-3-one 1,1-dioxide derivatives useful as anxiolytic and antihypertensive agents. Specifically claimed is 2-(4-(2,3-dihydro-1,4-benzodiox-2-yl)methylamino)butyl)-1,2-benzisothiazol-3(2H)-one 1,1-dioxide.

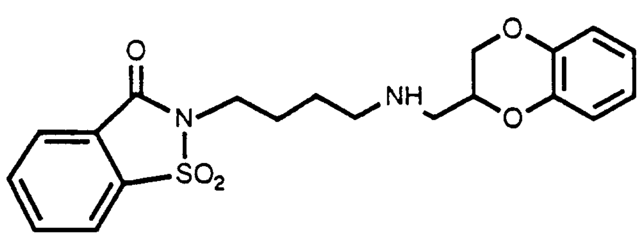

Neth 6,407,012 claims compounds of general formula (III), in which R, R1 and R2 are H, halogen, (1-6C) alkyl, or (1-6C) O-alkyl and n is an integer 2-6, as calming, hypnotic and hypotensive agents. Preferred among these structures are compounds in which $R^1$ and $R^2$ are oxygen-containing substituents. Jpn. Kokai

Tokkyo Koho JP 58,219,114 claims similar compounds in which the two oxygen substituents in the phenoxy moiety are joined by a methylene, ethylene, or propylene bridge.

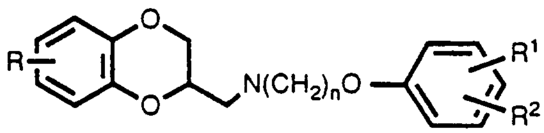

(III)

European Patent Application EP 175,541 discloses a series of aminoalkoxybenzopyranones (IV), useful as antipsychotic and anxiolytic agents, in which $R^1$ is aryl or heteroaryl piperazinyl or piperidinyl, R is hydrogen, lower alkyl, trifluoromethyl, or lower alkoxy, and n is an integer from 2 to 5.

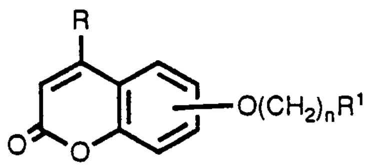

(IV)

## Description of the Invention

In accordance with this invention, there is provided a group of novel compounds of the formula:

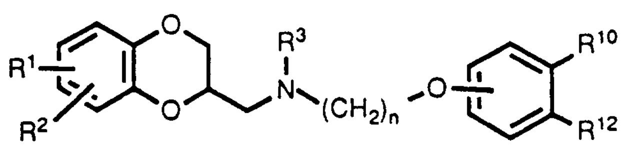

wherein

- $R^1$ and $R^2$ are, independently, hydrogen, alkyl of 1 to 6 carbon atoms, alkoxy of 1 to 6 carbon atoms, aralkoxy of 7 to 12 carbon atoms, alkanoyloxy of 2 to 6 carbon atoms, hydroxy, halo, amino, mono- or dialkylamino in which each alkyl group contains from 1 to 6 carbon atoms, alkanamido of 2 to 6 carbon atoms, or sulfonamido, or $R^1$ and $R^2$ together form methylenedioxy, ethylenedioxy, or propylenedioxy;
- $R^3$ is hydrogen or alkyl of 1 to 6 carbon atoms;
- n is one of the integers 2, 3 or 4;
- $R^{12}$ is $NR^4R^5$ in which $R^4$ and $R^5$ are, independently, hydrogen, alkyl of 1 to 6 carbon atoms, cycloalkyl of 4 to 7 carbon atoms, alkanoyl of 2 to 6 carbon atoms, aroyl of 7 to 12 carbon atoms, alkylsulfonyl of 1 to 6 carbon atoms or arylsulfonyl of 6 to 10 carbon atoms, or $R^4$ and $R^5$ together form a 3-7 membered polymethylene ring;
- $R^{10}$ is hydrogen or, when taken with $R^{12}$, $R^{10}$ and $R^{12}$ complete the six membered ring -

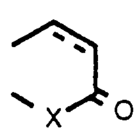

in which the dotted line represents optional unsaturation and X is O or $NR^8$, in which $R^8$ is hydrogen or alkyl of 1 to 6 carbon atoms;

or a pharmaceutically acceptable salt thereof.

Within the group of compounds described above are those of the formula:

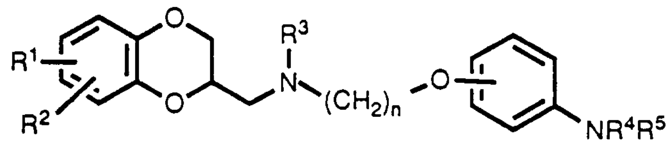

wherein

| | |
|---|---|
| $R^1$ and $R^2$ | are, independently, hydrogen, alkyl of 1 to 6 carbon atoms, alkoxy of 1 to 6 carbon atoms, aralkoxy of 7 to 12 carbon atoms, alkanoyloxy of 2 to 6 carbon atoms, hydroxy, halo, amino, mono- or dialkylamino in which each alkyl group contains from 1 to 6 carbon atoms, alkanamido of 2 to 6 carbon atoms, or sulfonamido, or $R^1$ and $R^2$ together form methylenedioxy, ethylenedioxy, or propylenedioxy; |
| $R^3$ | is hydrogen or alkyl of 1 to 6 carbon atoms; |
| n | is one of the integers 2, 3 or 4; |
| $R^4$ and $R^5$ | are, independently, hydrogen, alkyl of 1 to 6 carbon atoms, cycloalkyl of 4 to 7 carbon atoms, alkanoyl of 2 to 6 carbon atoms, aroyl of 7 to 12 carbon atoms, alkylsulfonyl of 1 to 6 carbon atoms or arylsulfonyl of 6 to 10 carbon atoms, or $R^4$ and $R^5$ together form a 3-7 membered polymethylene ring; |

or a pharmaceutically acceptable salt thereof.

Of these compounds, the preferred members are those in which $R^1$ and $R^2$ are hydrogen, fluoro, hydroxy, alkoxy of 1 to 6 carbon atoms or alkanoyloxy of 2 to 6 carbon atoms, or together form a methylenedioxy, ethylenedioxy or propylenedioxy ring; $R^4$ and $R^5$ are hydrogen or alkyl of 1 to 6 carbon atoms; n and $R^3$ are defined as above and the connection from the oxygen to the anilino moiety is in the meta position.

In addition, the genus of this invention embraces the compounds of the formula:

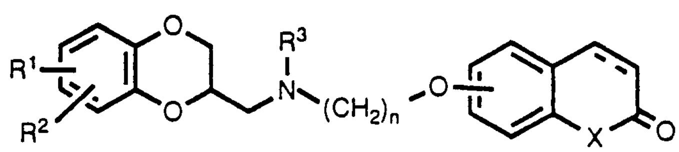

wherein

the dotted line represents optional unsaturation;

| | |
|---|---|
| $R^1$ and $R^2$ | are, independently, hydrogen, alkyl of 1 to 6 carbon atoms, alkoxy of 1 to 6 carbon atoms, aralkoxy of 7 to 12 carbon atoms, alkanoyloxy of 2 to 6 carbon atoms, hydroxy, halo, amino, mono- or dialkylamino in which each alkyl group has 1 to 6 carbon atoms, alkanamido of 2 to 6 carbon atoms or sulfonamido or $R^1$ and $R^2$ together are methylenedioxy, ethylenedioxy or propylenedioxy; |
| $R^3$ | is hydrogen or alkyl of 1 to 6 carbon atoms; |
| n | is one of the integers 2, 3 or 4; |
| X | is O or $NR^8$, in which $R^8$ is hydrogen or alkyl of 1 to 6 carbon atoms; |

or a pharmaceutically acceptable salt thereof.

Of these compounds, the preferred members are those in which $R^1$ and $R^2$ are, independently, fluoro, hydrogen, hydroxy, alkoxy of 1 to 6 carbon atoms or alkanoyloxy of 2 to 6 carbon atoms, or together form a methylenedioxy, ethylenedioxy or propylenedioxy ring; n, X, and $R^3$ are defined as above and the connection from the oxygen to the coumarin (X = O) or carbostyril (X = $NR^8$) moiety is in the 7 position.

Most preferred are those members in which $R^1$ and $R^2$ are located in the 6 and/or 7 positions of the benzodioxan and are defined as hydrogen, fluoro, hydroxy, alkoxy of 1 to 4 carbon atoms or alkanoyloxy of 2 to 4 carbon atoms, or together $R^1$ and $R^2$ form a methylenedioxy, ethylenedioxy or propylenedioxy ring; $R^3$ is hydrogen or alkyl of 1 to 4 carbon atoms; n is 3; where $R^{12}$ is $NR^4R^5$ and $R^{10}$ is hydrogen, $R^4$ and $R^5$ are hydrogen and the connection from oxygen to the aniline moiety is meta, and when $R^{10}$ and $R^{12}$ are taken together, X is oxygen and the connection from oxygen to the coumarin moiety is at the 7 position and

the dashed line is replaced with a solid line to represent unsaturation. In all the compounds of this invention, the preferred configuration of the benzodioxan methanamine is S. Throughout this application, the name of a product of this invention, where the absolute configuration of the benzodioxan methanamine is not indicated, is intended to embrace the R and S isomers, as well as a mixture of the R and S isomers.

The pharmaceutically acceptable salts are those derived from such organic and inorganic acids as: acetic, lactic, citric, tartaric, succinic, maleic, malonic, hydrochloric, hydrobromic, phosphoric, nitric, sulfuric, methanesulfonic, and similarly known acceptable acids.

The compounds of this invention are prepared by conventional methods. For example, the appropriately substituted benzodioxan methanamine is combined with a suitable nitrophenoxyalkyl halide in the presence of an acid scavenger such as diisopropylethylamine in a solvent such as dimethylformamide and heated at 80-100 °C for 24 hours, followed by reduction of the nitro group with hydrogen over palladium on carbon (1). Alternatively, a benzodioxan methylhalide or tosylate may be combined with the appropriate aminoalkoxynitrobenzene under similar conditions and heated for an extended period, again followed by reduction with hydrogen over palladium on carbon (2). The amine component may also be combined with a suitably substituted aldehyde and a reducing agent such as sodium cyanoborohydride or with the appropriate acid chloride followed by reduction of the amide by an agent such as borane/THF. The anilino nitrogen may be alkylated, acylated, or alkyl- or arylsulfonylated by conventional methods; however, in some cases protection of the benzodioxan methanamine prior to the nitro group reduction may be desirable.

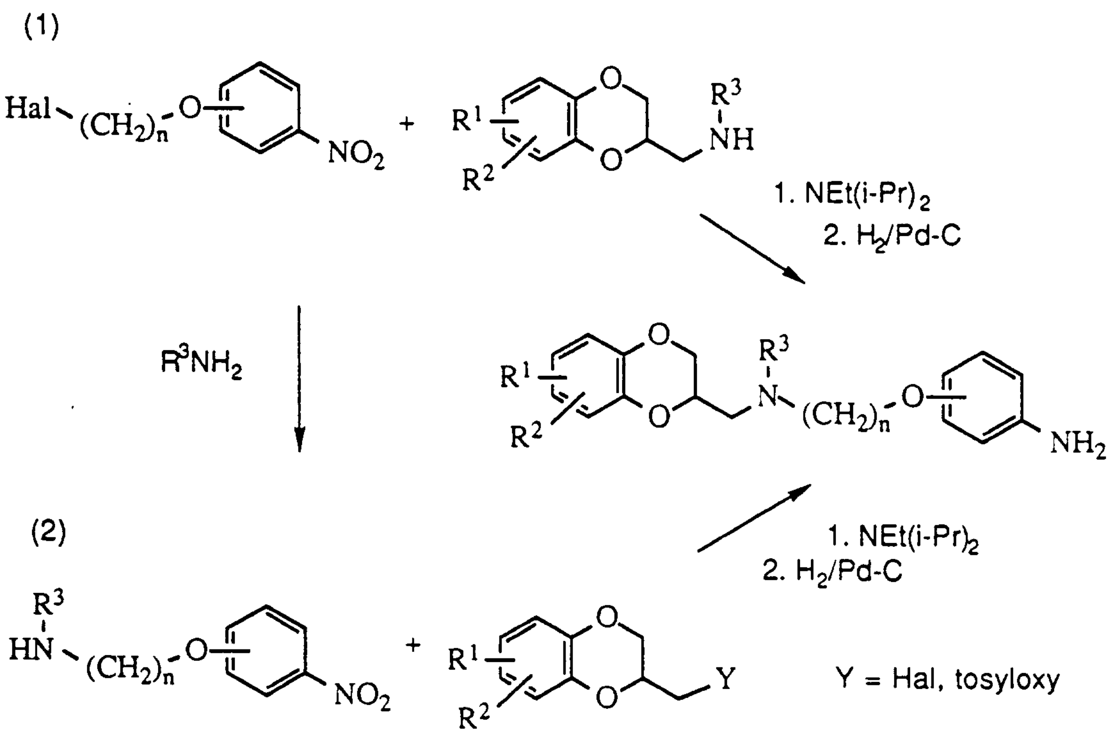


The nitrophenoxyalkyl halides appropriate for the above procedure are known compounds; the aminoalkoxynitrobenzenes may be readily prepared from them as shown above.

The appropriately substituted benzodioxan methanamine is combined with a suitable alkyl halide in the presence of an acid scavenger such as diisopropylethylamine in a solvent such as dimethylformamide and heated at 80-100 °C for 24 hours (3). Alternatively, a benzodioxan methylhalide or tosylate may be combined with the `appropriate aminoalkoxycoumarin or aminoalkoxycarbostyril under similar conditions and heated for an extended period (4). The amine component may also be combined with a suitably substituted aldehyde and a reducing agent such as sodium cyanoborohydride, or with the appropriate acid chloride followed by reduction by an agent such as borane/THF.

(3)

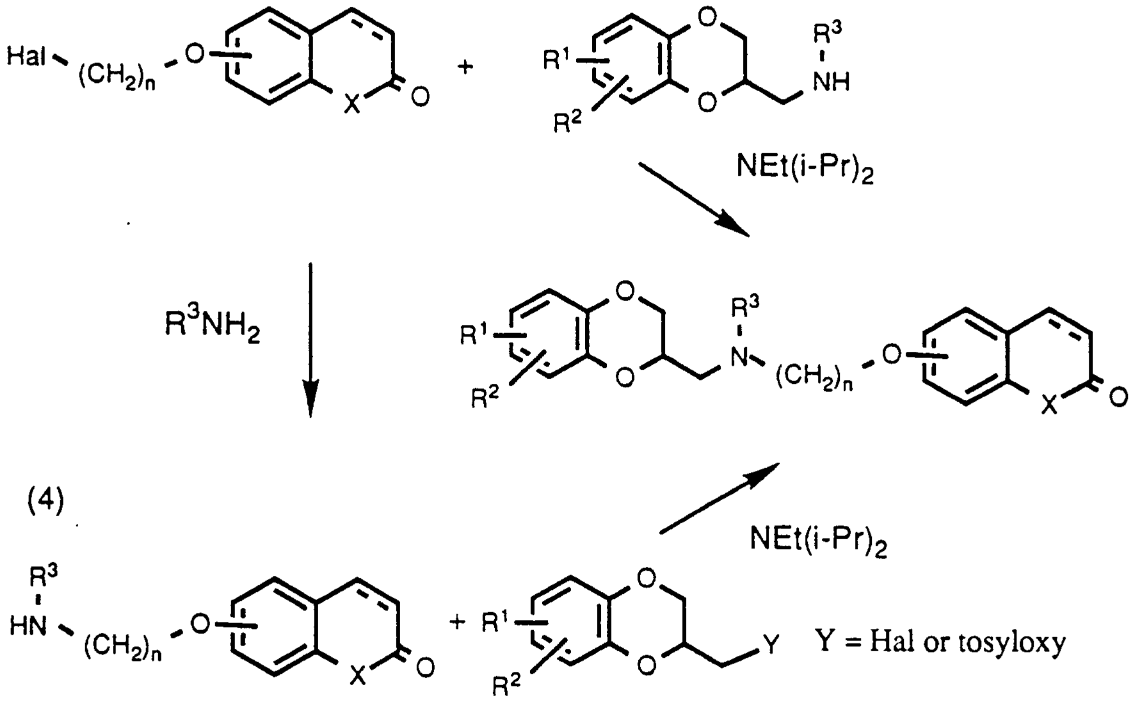


(4)

The haloalkoxycoumarins and carbostyrils appropriate for the above procedure are known compounds; the aminoalkoxycoumarins and carbostyrils may be readily prepared from them as shown above.

The aldehydes and carboxylic acid chlorides may also be readily prepared by one schooled in the art. The benzodioxan methanamine themselves are known compounds, or they can readily be derived from the appropriate salicylaldehyde by the procedure illustrated below. The benzodioxan methanamines may be resolved into their enantiomers by conventional methods or, preferably, they may be prepared directly by substitution of (2R)-(-)-glycidyl 3-nitrobenzenesulfonate (for the S benzodioxan methanamine) or (2S)-(+)-glycidyl 3-nitrobenzenesulfonate (for the R enantiomer) in place of epichlorohydrin in the procedure below.

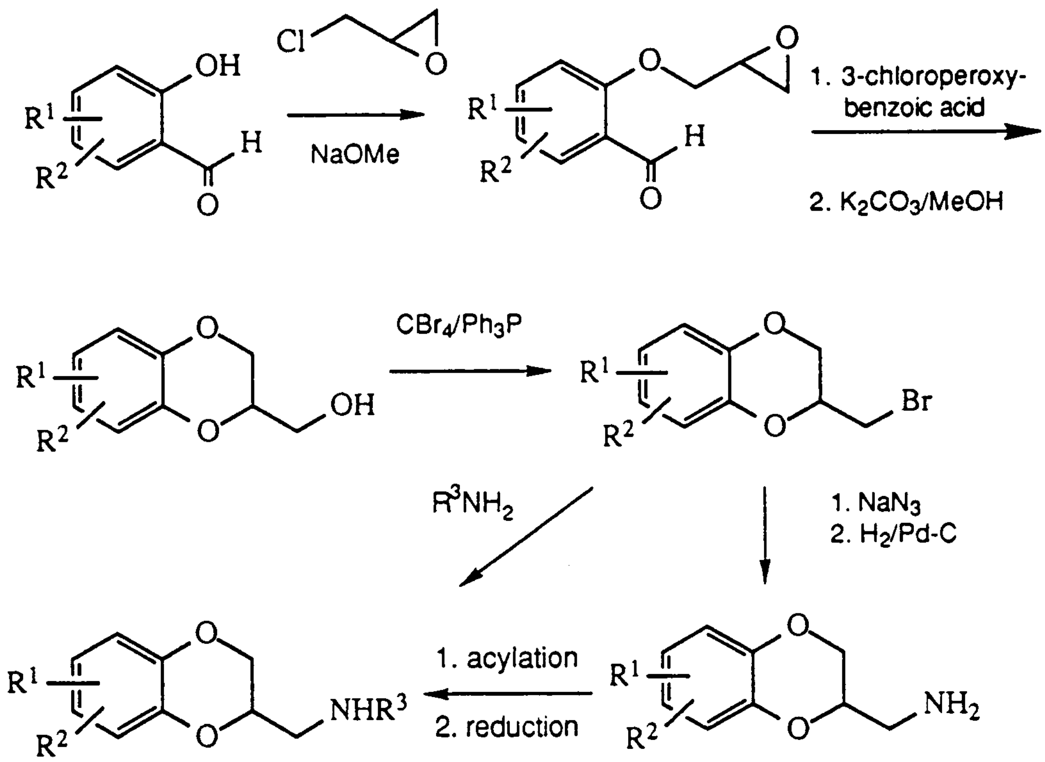


The compounds of this invention are intermediates or dopamine autoreceptor agonists; that is, they serve to modulate the synthesis and release of the neurotransmitter dopamine. They are thus useful for treatment of disorders of the dopaminergic system, such as schizophrenia, Parkinson's disease and Tourette's syndrome. Such agents are partial agonists at the postsynaptic dopamine $D_2$ receptor and are consequently of use in the treatment of drug addiction. Certain of the compounds of the invention also possess high affinity for serotonin 5-$HT_{1A}$ receptors and consequently, like the serotonergic agent buspirone, they are useful as antidepressant and anxiolytic agents for the treatment of a variety of central nervous system disorders such as depression, anxiety, sleep and eating disorders, sexual dysfunction, and related problems.

The effect of the compounds of the invention on the synthesis of dopamine was established by the method of Walters and Roth, Naunyn-Schmiedeberg's Arch. Pharmacol. 296:5-14, 1976, in which rats (male, Sprague-Dawley, Charles River, 200-350 g) were administered vehicle or test drug ten minutes prior to the administration of gamma butyrolactone (GBL; 750 mg/kg, ip to inhibit dopaminergic impulse flow) and 20 minutes prior to NSD-1015 (100 mg/kg, ip to prevent the conversion of dopa to dopamine). Thirty minutes after NSD-1015 all rats were decapitated and the nucleus accumbens and the striatum were removed for analysis. Following perchloric acid extraction of the tissue, the extracts were placed over alumina columns to collect and concentrate dopa and other catechols. This eluate was then subjected to HPLC analysis using electrochemical detection to quantify the levels of dopa present. Dopamine autoreceptor agonists, under the conditions used above, inhibit dopa accumulation. The results of this testing with compounds representative of this invention are reported below as % inhibition of dopa accumulation at 10 mg/kg, sc in either limbic (L) or striatal (S) brain tissue.

The antipsychotic activity of the compounds of the invention was further established by a determination of the compounds' ability to reduce mouse locomotor activity according to the method of Martin and Bendensky, J. Pharmacol. Exp. Therap. 229: 706-711, 1984, in which mice (male, CF-1, Charles River, 20-30 g) were injected with vehicle or various doses of each drug and locomotor activity was measured for 30 minutes using automated infrared activity monitors (Omnitech - 8 x 8 inch open field) located in a darkened room. $ED_{50}$'s were calculated from the horizontal activity counts collected from 10 to 20 minutes after dosing using a nonlinear regression analysis with inverse prediction. The results of this test with compounds of the invention are reported below.

Affinity for the dopamine $D_2$ receptor was established by the standard experimental test procedure of Fields, et al., Brain Res., 136, 578 (1977) and Yamamura et al., eds., Neurotransmitter Receptor Binding, Raven Press, N.Y. (1978) wherein homogenized limbic brain tissue is incubated with $^3H$-spiroperidol and

various concentrations of test compound, filtered and washed and shaken with Hydrofluor scintillation cocktail (National Diagnostics) and counted in a Packard 460 CD scintillation counter. The results of this testing with compounds representative of this invention are also given below.

Affinity for the serotonin 5-$HT_{1A}$ receptor was established by testing the claimed compound's ability to displace [$^3$H] 8-OHDPAT (dipropylaminotetralin) from the 5-$HT_{1A}$ serotonin receptor following the procedure of Hall et al., J. Neurochem. 44, 1685 (1985). This procedure is employed to analogize this property of the claimed compounds with that of buspirone, which is a standard for anxiolytic activity, and, like the compounds of this invention, displays potent affinity for the 5-$HT_{1A}$ serotonin receptor subtype. The anxiolytic activity of buspirone is believed to be, at least partially, due to its 5-$HT_{1A}$ receptor affinity (Vander Maclen et al., Eur. J. Pharmacol. 1986, 129 (1-2) 133-130).

The results of the standard experimental test procedures described in the preceding four paragraphs were as follows:

| Compound | Dopa Accumulation (% inhib. @ 10 mg/kg, sc) | Hypolocomotion ($ED_{50}$ mg/kg, ip) | Receptor Affinities ($IC_{50}$ (nM) or % @ ( ) μM) D2 | 5-$HT_{1A}$ |
|---|---|---|---|---|
| Example 1 | 33 (L)/46(S) | 0.38 | 525 nM | 4 nM |
| Example 2 | -22 (L) | | | |
| Example 3 | 64 (L)/66 (S) | 0.16/0.15 | 51 nM | 8 nM |
| Example 4 | 70 (L)/71 (S) | 0.08 | 50/45 nM | 15 nM |
| Example 5 | 31 (L)/30 (S) | | | |
| Example 6 | 23 (L)/30 (S) | | | |
| Example 7 | 27 (L)/19 (S) | 1.34 | 70% (1.0) | 72 nM |
| Example 8 | 42.9 (L) | | 48% (1.0) | 16 nM |

| Compound | Dopa Accumulation (% inhib. @ 10 mg/kg, sc) | Hypolocomotion ($ED_{50}$ mg/kg, ip) | Receptor Affinities ($IC_{50}$ (nM) or % @ ( ) μM) $D_2$ | $5\text{-}HT_{1A}$ |
|---|---|---|---|---|
| Example 9 | | 0.19 | | 100% (0.1) |
| Example 10 | 70.9 (L) | 1.8 | 1621 nM | 117 nM |
| Example 11 | 13.0 (L) | | | |
| Example 12 | 17.4 (L) | | | 100% (0.1) |
| Example 13 | 9.1 (L) | | | 89% (0.1) |
| Example 14 | 53.7 (L) | 10.5 (sc) | | 22% (0.1) |
| Example 15 | 7.6 (L) | | 0% (1.0) | 20% (0.1) |
| Example 16 | 59.9 (L) | 1.4 | 387 nM | 12 nM |
| Example 17 | 28 (L) | | 25% (1.0) | 92% (0.1) |
| Example 18 | 10.6 (L) | | | 45% (0.1) |
| Example 19 | 11.5 (L) | | 85% (1.0) | 82% (0.1) |
| Example 20 | 39 (L)/1 (S) | 0.58 | 112 nM | 0.5 nM |
| Example 21 | -12 (L) | | | |
| Example 23 | 20 (L) | | | |
| Example 24 | 60 (L)/18 (S) | 1.62/2.04 | 337/730 nM | 112 nM |
| Example 25 | 76 (L)/73 (S) | 0.19 | 100% (1.0) | 6 nM |
| Example 26 | 1.5 (L)/51.3 (S) | | | |
| Example 27 | 50 (L) | 3.5 | | 104 nM |
| Example 28 | 21.7 (L) | 1.6 | 66% (1.0) | |
| Example 29 | 24.0 | | | |
| Example 30 | 37.7 (L) | 2.8 | | 78% (0.1) |
| Example 31 | 61.6 (L) | 1.6 | | 82 nM |
| Example 32 | 6.9 (L)/18 (L) | | | 100% (0.1) |
| Example 33 | 19.5 | | | 66% (0.1) |
| Example 34 | 9.2 | | | 23% (0.1) |
| Example 35 | 25.9 (L) | | | 55% (0.1) |
| Example 36 | 7.3 (L) | | 35% (1.0) | |
| Example 37 | 9.4 (L) | | 61% (1.0) | 96% (0.1) |
| Example 38 | 23 (L) | | 21% (1.0) | 67% (0.1) |

| Compound | Dopa Accumulation (% inhib. @ 10 mg/kg, sc) | Hypolocomotion ($ED_{50}$ mg/kg, ip) | Receptor Affinities ($IC_{50}$ (nM) or % @ ( ) μM) $D_2$ | $5\text{-}HT_{1A}$ |
|---|---|---|---|---|
| Example 39 | 25 (L) | | 92% (1.0) | 95% (0.1) |
| Example 40 | 48 (L) | 1.8 | 81% (1.0) | |
| Example 41 | 6 (L) | | 897 nM | 94% (0.1) |

Hence, the compounds of this invention have a pronounced effect on the synthesis of the neurotransmitter dopamine and thus are useful in the treatment of dopaminergic disorders such as schizophrenia, Parkinson's disease, Tourette's syndrome and drug addiction. It should be noted that the 5-methoxy bearing compounds of Example 2 and Example 21 did not inhibit dopa conversion to dopamine. In effect these compounds were inactive. However, they are metabolized to the corresponding 5-hydroxy derivatives which are active inhibitors of dopa conversion, as shown with the product of Example 6. Hence, the products of Example 2 and 21 are pro-drugs for the corresponding 5-hydroxy analogues. Certain compounds also demonstrated high affinity for both the serotonin $5\text{-}HT_{1A}$ and dopamine $D_2$ receptor subtypes, and are therefore useful in the treatment of multi-CNS disorders amenable to treatment with antipsychotic, antidepressant and anxiolytic agents. As such, the compounds of this invention are useful in relieving the symptoms of anxiety, depression and various psychoses by administration, orally or parenterally to a patient in need thereof.

Applicable solid carriers can include one or more substances which may also act as flavoring agents, lubricants, solubilizers, suspending agents, fillers, glidants, compression aids, binders or tablet-disintegrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form.

The dosage to be used in the treatment of a specific psychosis, state of depression or anxiety must be subjectively determined by the attending physician. The variables involved include the specific psychosis, degree of depression or state of anxiety and the size, age and response pattern of the patient.

The following examples illustrate the production of representative compounds of this invention.

## EXAMPLE 1

**N-[3-(3-Aminophenoxy)propyl]-2,3-dihydro-1,4-benzodioxin-2-methanamine**

2,3-Dihydro-1,4-benzodioxin-2-methanamine (2.52 g, 15.3 mmole), 3-(3-nitrophenoxy)propyl chloride (2.99 g, 13.9 mmole), diisopropylethylamine (13.75 ml, 79 mmole) and sodium iodide (2.31 g, 15.4 mmole) were combined in 200 ml of DMF and heated at 95 °C for 18.5 hours under a nitrogen atmosphere. The solvent was then removed in vacuum and the residue was column chromatographed on silica gel using 1% methanol/dichloromethane as eluant. The product-containing fractions (Rf = 0.75 on silica gel tlc with 5% methanol/dichloromethane) were combined and concentrated in vacuum to give 2.46 g of a brown oil. This oil was dissolved in 125 ml of methanol and 0.75 g of 10% palladium on carbon added, along with 2 ml of 4 N isopropanolic HCl. The mixture was hydrogenated at 50 psi on a Parr apparatus for 4 hours. The mixture was filtered through celite, concentrated in vacuum and the residue crystallized from isopropanol with another addition of 4 N HCl/isopropanol to give 1.63 g of title compound as a gray solid, dihydrochloride, m.p. 223-228 °C.

| Elemental Analysis for: $C_{18}H_{22}N_2O_3$ • 2 HCl | | | |
|---|---|---|---|
| Calcd: | C, 55,82; | H, 6.25; | N, 7.23 |
| Found: | C, 55.97; | H, 6.34; | N, 7.31 |

## EXAMPLE 2

**N-[3-(3-Aminophenoxy)propyl]-2,3-dihydro-5-methoxy-1,4-benzodioxin-2-methanamine**

2,3-Dihydro-5-methoxy-1,4-benzodioxin-2-methanamine hydrochloride (2.21 g, 9.54 mmole), 3-(3-nitrophenoxy)propyl chloride (1.95 g, 9.76 mmole), diisopropylethylamine (6.8 ml, 39 mmole) and sodium iodide (7.35 g, 49 mmole) were combined in 100 ml of DMF and heated at 80 - 100 °C for 3 days under a nitrogen atmosphere. The solvent was then removed in vacuum and the residue was column chromatographed on silica gel using first 30% ethyl acetate/petty ether and then 2.5% methanol/dichloromethane as eluant. The product-containing fractions were combined and concentrated in vacuum to give 2.04 g of nitrophenoxyalkyl derivative. This was dissolved in 200 ml of methanol/water, 0.36 g of 5% palladium on carbon and 3 ml of 4 N isopropanolic HCl added and the mixture hydrogenated at 50 psi on a Parr apparatus for 3 hours. The mixture was filtered through celite, concentrated in vacuum, and the residue crystallized from isopropanol to give 1.45 g of title compound as a white solid, dihydrochloride, quarter hydrate, m.p. 230-236 °C.

| Elemental Analysis for: $C_{19}H_{24}N_2O_4$ • 2 HCl • 1/4 $H_2O$ | | | |
|---|---|---|---|
| Calcd: | C, 54.10; | H, 6.33; | N, 6.64 |
| Found: | C, 54.47; | H, 6.50; | N, 6.32 |

## EXAMPLE 3

**N-[3-(3-Aminophenoxy)propyl]-2,3-dihydro-7-hydroxy-1,4-benzodioxin-2-methanamine**

2,3-Dihydro-7-benzyloxy-1,4-benzodioxin-2-methanamine (1.49 g, 5.49 mmole), 3-(3-nitrophenoxy)propyl chloride (1.13 g, 5.66 mmole), diisopropylethylamine (4.0 ml, 23 mmole) and sodium iodide (4.18 g, 27.9 mmole) were combined in 150 ml of DMF and heated at 80 - 100 °C for 24 hours under a nitrogen atmosphere. The solvent was then removed in vacuum and the residue was column chromatographed on silica gel using a gradient elution commencing with dichloromethane and ending with 1.5% methanol/dichloromethane as eluant. The product-containing fractions were combined and concentrated in vacuum to give 0.82 g of nitrophenoxyalkyl derivative as a brown oil. This was dissolved in 75 ml of ethanol, and 0.20 g of 5% palladium on carbon added and the mixture hydrogenated at 50 psi on a Parr apparatus for 24 hours. The mixture was filtered through celite, concentrated in vacuum, and the residue crystallized

from 50 ml of isopropanol with the addition of 3 ml of 4 N HCl/isopropanol to give 0.47 g of title compound as a beige solid, dihydrochloride, quarter hydrate, m.p. 173 °C.

| Elemental Analysis for: $C_{18}H_{22}N_2O_4$ • 2 HCl • 1/4 $H_2O$ | | | |
|---|---|---|---|
| Calcd: | C, 53.01; | H, 6.05; | N, 6.87 |
| Found: | C, 53.15; | H, 6.01; | N, 6.56 |

2,3-Dihydro-6-methyl-7-hydroxy-N-[3-(3-aminophenoxy)propyl]-1,4-benzodioxin-2-methanamine was produced in similar fashion via the reaction of 2,3-dihydro-6-methyl-7-hydroxy-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 230-232 °C) via addition of 4N HCl/IPA to an ethanolic solution of the free base, followed by addition of three volumes of ether.

| Elemental Analysis for: $C_{19}H_{24}N_2O_4$ • 2 HCl | | | |
|---|---|---|---|
| Calcd: | C, 54.68; | H, 6.28; | N, 6.71 |
| Found: | C, 54.64; | H, 6.30; | N, 6.48 |

## EXAMPLE 4

**(S)-N-[3-(3-Aminophenoxy)propyl]-2,3-dihydro-7-hydroxy-1,4-benzodioxin-2-methanamine**

(S)-2,3-Dihydro-7-hydroxy-1,4-benzodioxin-2-methanamine (2.37 g, 13.1 mmole), 3-(3-nitrophenoxy)-propyl chloride (2.18 g, 10.1 mmole), diisopropylethylamine (9.0 ml, 52 mmole) and sodium iodide (2.84 g, 19 mmole) were combined in 175 ml of DMF and heated at 97 °C for 25 hours under a nitrogen atmosphere. The solvent was then removed in vacuum and the residue was column chromatographed on silica gel using a gradient elution commencing with 0.5% methanol/dichloromethane and ending with 1.5% methanol/dichloromethane as eluant. The product-containing fractions were combined and concentrated in vacuum to give 2.87 g of nitrophenoxyalkyl derivative as a purple semi-solid. This was dissolved in 125 ml of ethanol, and 0.30 g of 10% palladium on carbon added and the mixture hydrogenated at 50 psi on a Parr apparatus for 24 hours. The mixture was filtered through celite, concentrated in vacuum, and the residue column chromatographed on silica gel using 1% methanol/dichloromethane. The product-containing fractions (Rf = 0.26 with 5% methanol/dichloromethane on silica gel) were combined and evaporated in vacuum and the residue crystallized from isopropanol with the addition of 4 ml of 4 N HCl/isopropanol to give 1.1 g of title compound as a tan solid, monohydrochloride, m.p. 182-185 °C, $[\alpha]_D^{25}$ = -43.4 °.

| Elemental Analysis for: $C_{18}H_{22}N_2O_4$ • 2 HCl | | | |
|---|---|---|---|
| Calcd: | C, 53.61; | H, 6.00; | N, 6.95 |
| Found: | C, 53.46; | H, 6.13; | N, 6.83 |

## EXAMPLE 5

**N-[3-(3-Aminophenoxy)propyl]-2,3-dihydro-6-hydroxy-1,4-benzodioxin-2-methanamine**

2,3-Dihydro-6-hydroxy-1,4-benzodioxin-2-methanamine (2.37 g, 13.1 mmole), 3-(3-nitrophenoxy)propyl chloride (2.20 g, 10.2 mmole), diisopropylethylamine (9.0 ml, 53.3 mmole) and sodium iodide (2.81 g, 18.8 mmole) were combined in 175 ml of DMF and heated at 80 - 100 °C for 2 days under a nitrogen atmosphere. The solvent was then removed in vacuum and the residue was column chromatographed on silica gel using a gradient elution commencing with 0.5% methanol/dichloromethane and ending with 1.5% methanol/dichloromethane as eluant. The product-containing fractions were combined and concentrated in vacuum to give 3.70 g of nitrophenoxyalkyl derivative as a brown oil. 3.19 g of this material was dissolved in 75 ml of ethanol, and 0.50 g of 10% palladium on carbon and 7.0 ml of 4 N HCl/isopropanol added and the

mixture hydrogenated at 50 psi on a Parr apparatus for 7 days, with two more. additions of catalysts on days 2 and 6. The mixture was filtered through celite, concentrated in vacuum, and the residue crystallized from 75 ml of isopropanol to give 0.95 g of title compound as a beige solid, dihydrochloride, half hydrate, m.p. 242-245 °C.

| Elemental Analysis for: $C_{18}H_{22}N_2O_4$ • 2 HCl • 1/2 $H_2O$ | | | |
|---|---|---|---|
| Calcd: | C, 52.44; | H, 6.11; | N, 6.79 |
| Found: | C, 52.67; | H, 6.06; | N, 6.80 |

**EXAMPLE 6**

**N-[3-(3-Aminophenoxy)propyl]-2,3-dihydro-5-hydroxy-1,4-benzodioxin-2-methanamine**

N-[3-(3-Aminophenoxy)propyl]-2,3-dihydro-5-methoxy-1,4-benzodioxin-2-methanamine dihydrochloride quarter hydrate (0.79 g, 1.9 mmole), prepared in Example 2 above, was dissolved in 26.5 ml of 48% HBr and refluxed under nitrogen for 23 hours. Upon cooling, the mixture was diluted to 250 ml with water and carefully neutralized with solid sodium bicarbonate. It was then extracted with two 200 ml portions of 3:1 dichloromethane/isopropanol and the combined extracts were washed with saturated aqueous sodium chloride, dried over magnesium sulfate, filtered and concentrated to dryness in vacuum. The residue was column chromatographed on silica gel using 5% methanol/dichloromethane to which 1 ml/liter aqueous ammonia had been added. The product-containing fractions were combined, concentrated in vacuum, and the residue crystallized from 20 ml of isopropanol with the addition of 4 ml 4 N HCl/isopropanol to give 0.33 g of the title compound (m.p. 183-190 °C) as a beige solid, dihydrochloride.

| Elemental Analysis for: $C_{18}H_{22}N_2O_4$ • 2 HCl | | | |
|---|---|---|---|
| Calcd: | C, 53.61; | H, 6.00; | N, 6.95 |
| Found: | C, 53.83; | H, 6.33; | N, 6.55 |

**EXAMPLE 7**

**(R)-N-[3-(3-Aminophenoxy)propyl]-2,3-dihydro-7-hydroxy-1,4-benzodioxin-2-methanamine**

(R)-2,3-Dihydro-7-hydroxy-1,4-benzodioxin-2-methanamine (5.63 g, 31.0 mmole), 3-(3-nitrophenoxy)-propyl chloride (6.27 g, 29.1 mmole), diisopropylethylamine (15.5 ml, 89 mmole) and sodium iodide (4.25 g, 28.4 mmole) were combined in 200 ml of DMF and heated at 77 °C for 24 hours under a nitrogen atmosphere. The solvent was then removed in vacuum and the residue was column chromatographed on silica gel using a gradient elution commencing with 0.5% methanol/dichloromethane and ending with 2.5% methanol/dichloromethane as eluant. The product-containing fractions were combined and concentrated in vacuum to give 3.29 g of nitrophenoxyalkyl derivative as a light brown oil. This was dissolved in 100 ml of ethanol, and 0.75 g of 10% palladium on carbon added and the mixture hydrogenated at 50 psi on a Parr apparatus for 3 days. The mixture was filtered through celite, concentrated in vacuum, and the residue crystallized from 70 ml of isopropanol with the addition of 10 ml of 4 N HCl/IPA to give 2.13 g of title compound as a tan solid, dihydrochloride, m.p. 181-190 °C, $[\alpha]_D^{25}$ = +42.7° (MeOH).

| Elemental Analysis for: $C_{18}H_{22}N_2O_4$ • 2 HCl | | | |
|---|---|---|---|
| Calcd: | C, 53.61; | H, 6.00; | N, 6.95 |
| Found: | C, 53.36; | H, 5.77; | N, 7.00 |

## EXAMPLE 8

### N-[3-(3-Aminophenoxy)propyl]-2,3-dihydro-7-hydroxy-1,4-benzodioxin-2-methanamine

2,3-Dihydro-7-methoxy-1,4-benzodioxin-2-methanamine (3.8 g, 20 mmole), 3-(3-nitrophenoxy)propyl bromide (5.2 g, 20 mmole), and diisopropylethylamine (2.6 g, 20 mmole) were combined in 200 ml of DMF and heated at 100 °C for 24 hours under a nitrogen atmosphere. The solvent was then removed in vacuum and the residue column chromatographed on 100 g of silica gel using first dichloromethane, then chloroform, and finally 2% methanol/chloroform as eluant. The product-containing fractions were combined and concentrated in vacuum to give 3.25 g of a yellow solid. A portion (1.25 g, 3.3 mmole) of this material was dissolved in 200 ml of ethanol and 0.50 g of 10% palladium on carbon added, along with 4 ml of 4 N isopropanolic HCl. The mixture was hydrogenated at 50 psi on a Parr apparatus for 24 hours. The mixture was filtered through celite, concentrated in vacuum and the residue crystallized from 75 ml of isopropanol to give 1.0 g of title compound as a white solid, dihydrochloride, m.p. 210-212 °C.

| Elemental Analysis for: $C_{19}H_{24}N_2O_4$ • 2 HCl | | | |
|---|---|---|---|
| Calcd: | C, 54.68; | H, 6.28; | N, 6.71 |
| Found: | C, 54.34; | H, 6.10; | N, 6.71 |

2,3-Dihydro-6-methyl-7-methoxy-N-[3-(3-aminophenoxy)propyl]-1,4-benzodioxin-2-methanamine was produced in the same manner via the reaction of 2,3-dihydro-6-methyl-7-methoxy-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 237-238 °C) as described above.

| Elemental Analysis for: $C_{20}H_{26}N_2O_4$ • 2 HCl | | | |
|---|---|---|---|
| Calcd: | C, 55.69; | H, 6.54; | N, 6.49 |
| Found: | C, 55.68; | H, 6.64; | N, 6.19 |

## EXAMPLE 9

### (S)-N-[3-(3-Aminophenoxy)propyl]-2,3-dihydro-1,4-benzodioxin-2-methanamine

(S)-2,3-Dihydro-1,4-benzodioxin-2-methanamine (3.5 g, 21 mmole), 3-(3-nitrophenoxy)propyl bromide (5.5 g, 21 mmole) and diisopropylethylamine (2.7 g, 21 mmole) were combined in 100 ml of DMF and heated at 80 - 90 °C for 24 hours under a nitrogen atmosphere. The solvent was then removed in vacuum and the residue was redissolved in 350 ml of dichloromethane and washed with 250 ml portions of saturated aqueous sodium bicarbonate solution and saturated sodium chloride solution, dried over sodium sulfate, filtered and evaporated to dryness in vacuum. The residue was column chromatographed on 100 g silica gel with a gradient elution commencing with chloroform and ending with 2% methanol in chloroform. The product-containing fractions were combined and concentrated in vacuum to give 3.85 g of an orange oil. A portion (1.25 g, 3.6 mmole) of this oil was dissolved in 150 ml of methanol and 0.50 g of 10% palladium on carbon added, along with 3 ml of 4 N isopropanolic HCl. The mixture was hydrogenated at 50 psi on a Parr apparatus for 24 hours. The mixture was filtered through celite and concentrated and the residue crystallized from 50 ml of isopropanol to give 1.1 g of title compound as a white solid, dihydrochloride, m.p. 245-247 °C.

| Elemental Analysis for: $C_{18}H_{22}N_2O_3$ • 2 HCl | | | |
|---|---|---|---|
| Calcd: | C, 55.82; | H, 6.25; | N, 7.23 |
| Found: | C, 55.61; | H, 6.17; | N, 7.10 |

## EXAMPLE 10

**N-[3-(3-Aminophenoxy)propyl]-2,3-dihydro-6,7-methylenedioxy-1,4-benzodioxin-2-methanamine**

2,3-Dihydro-6,7-methylenedioxy-1,4-benzodioxin-2-methanamine (3.59 g, 17 mmole), 3-(3-nitrophenoxy)propyl bromide (4.42 g, 17 mmole), diisopropylethylamine (14.8 ml, 85 mmole) and sodium iodide (2.80 g, 18.7 mmole) were combined in 200 ml of DMF and heated at 95 °C overnight under a nitrogen atmosphere. The solvent was then removed in vacuum and the residue triturated with hot methanol to produce a yellow solid. This was suspended in chloroform and washed with saturated aqueous sodium bicarbonate and the organic phase dried with magnesium sulfate, filtered and concentrated in vacuum. The residue was column chromatographed on silica gel using 5% methanol/chloroform as eluant. The product-containing fractions were combined and concentrated in vacuum to give 3.80 g of a yellow oil. A portion (1.8 g, 4.6 mmole) of this oil was dissolved in 50% methanol-water and 0.49 g of 10% palladium on carbon added, along with 1.15 ml of 4 N isopropanolic HCl. The mixture was hydrogenated at 50 psi on a Parr apparatus overnight, filtered through celite, concentrated in vacuum and the residue recrystallized from ethanol to give 0.62 g of title compound as a gray solid, dihydrochloride, m.p. 211-212 °C.

| Elemental Analysis for: $C_{19}H_{22}N_2O_5$ • 2 HCl | | | |
|---|---|---|---|
| Calcd: | C, 52.91; | H, 5.61; | N, 6.49 |
| Found: | C, 53.10; | H, 5.71; | N, 6.34 |

(S)-2,3-Dihydro-6,7-methylenedioxy-N-[3-(3-aminophenoxy)propyl]-1,4-benzodioxin-2-methanamine was produced in the same manner via the reaction of (S)-2,3-dihydro-6,7-methylenedioxy-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 229-231 °C) as described above.

| Elemental Analysis for: $C_{19}H_{22}N_2O_5$ • 2 HCl | | | |
|---|---|---|---|
| Calcd: | C, 52.91; | H, 5.61; | N, 6.49 |
| Found: | C, 52.69; | H, 5.52; | N, 6.43 |

(R)-2,3-Dihydro-6,7-methylenedioxy-N-[3-(3-aminophenoxy)propyl]-1,4-benzodioxin-2-methanamine was produced in the same manner via the reaction of (R)-2,3-dihydro-6,7-methylenedioxy-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 221-223 °C, $[\alpha]_D^{25}$ MeOH = +44.6) as described above.

| Elemental Analysis for: $C_{19}H_{22}N_2O_5$ • 2 HCl | | | |
|---|---|---|---|
| Calcd: | C, 52.91; | H, 5.61; | N, 6.49 |
| Found: | C, 52.76; | H, 5.93; | N, 6.45 |

## EXAMPLE 11

**N-[3-(3-Aminophenoxy)propyl]-2,3-dihydro-8-methoxy-1,4-benzodioxin-2-methanamine**

2,3-Dihydro-8-methoxy-1,4-benzodioxin-2-methanamine (3.8 g, 20 mmole), 3-(3-nitrophenoxy)propyl bromide (5.2 g, 20 mmole), and diisopropylethylamine (2.6 g, 20 mmole) were combined in 150 ml of DMF and heated at 80 °C for 24 hours under a nitrogen atmosphere. The solvent was then removed in vacuum and the residue column chromatographed on 100 g of silica gel using first dichloromethane, then chloroform, and finally 2% methanol/chloroform as eluant. The product-containing fractions were combined and concentrated in vacuum to give 5.5 g of a dark oil. This material was dissolved in 150 ml of methanol and 0.50 g of 10% palladium on carbon added, along with 10 ml of 4 N isopropanolic HCl. The mixture was hydrogenated at 55 psi on a Parr apparatus for 24 hours. The mixture was then filtered through celite, and

treated with charcoal while boiling on a hot plate. After a second filtration through celite, the mixture was brought to a boil on a hot plate, and the solvent gradually replaced with isopropanol The volume was then reduced to 75 ml and allowed to cool to give 2.2 g of title compound as a yellow solid, dihydrochloride, monohydrate, m.p. 200-205 °C.

| Elemental Analysis for: $C_{19}H_{24}N_2O_4$ • 2 HCl • $H_2O$ | | | |
|---|---|---|---|
| Calcd: | C, 52.42; | H, 6.48; | N, 6.44 |
| Found: | C, 52.39; | H, 6.38; | N, 6.48 |

**EXAMPLE 12**

**N-[3-(3-Aminophenoxy)propyl]-2,3-dihydro-8-hydroxy-1,4-benzodioxin-2-methanamine**

N-[3-(3-Aminophenoxy)propyl]-2,3-dihydro-8-methoxy-1,4-benzodioxin-2-methanamine dihydrochloride hydrate (1.2 g, 2.75 mmole), prepared in Example 11 above, was dissolved in 25 ml of 48% HBr and refluxed overnight under nitrogen. The mixture was diluted with water to 300 ml, carefully neutralized with solid sodium carbonate, and twice extracted with 200 ml of 3:1 dichloromethane/isopropanol. The combined extracts were washed with saturated aqueous sodium chloride, dried over sodium sulfate, filtered and concentrated in vacuum. The residue was crystallized from 50 ml of isopropanol with the addition of 2 ml of 4 N HCl/isopropanol to give 0.37 g of the title compound as a tan solid, dihydrochloride, quarter hydrate, m.p. 249-255 °C.

| Elemental Analysis for: $C_{18}H_{22}N_2O_4$ • 2 HCl • 1/4 $H_2O$ | | | |
|---|---|---|---|
| Calcd: | C, 53.01; | H, 6.05; | N, 6.87 |
| Found: | C, 52.89; | H, 6.18; | N, 6.76 |

**EXAMPLE 13**

**N-[3-(3-Aminophenoxy)propyl]-2,3-dihydro-7-chloro-1,4-benzodioxin-2-methanamine**

2,3-Dihydro-7-chloro-1,4-benzodioxin-2-methanamine (2.08 g, 8.81 mmole), 3-(3-nitrophenoxy)propyl bromide (2.09 g, 8.04 mmole) and diisopropylethylamine (7.0 ml, 40.2 mmole) were combined in 200 ml of DMF and heated at 80 °C for 3 days under a nitrogen atmosphere. The solvent was then removed in vacuum and the residue was column chromatographed on silica gel using 50 to 60% ethyl acetate/hexane as eluant. The product-containing fractions were combined and concentrated in vacuum to give 0.40 g of nitrophenoxyalkyl derivative. This was dissolved in 100 ml of methanol and 0.11 g of 10% palladium on carbon added, along with 2 ml of 4 N isopropanolic HCl. The mixture was hydrogenated at 50 psi on a Parr apparatus for 1 hour. The mixture was filtered through celite, concentrated in vacuum and the residue twice recrystallized from isopropanol to give 0.13 g of title compound as a white solid, dihydrochloride, three quarter hydrate, m.p. 216-226 °C.

| Elemental Analysis for: $C_{18}H_{21}ClN_2O_3$ • 2 HCl • 3/4 $H_2O$ | | | |
|---|---|---|---|
| Calcd: | C, 49.67; | H, 5.67; | N, 6.43 |
| Found: | C, 49.66; | H, 5.99; | N, 6.01 |

**EXAMPLE 14**

**N-[3-(3-Aminophenoxy)propyl]-2,3-dihydro-6,7-dimethoxy-1,4-benzodioxin-2-methanamine**

2,3-Dihydro-6,7-dimethoxy-1,4-benzodioxin-2-methanamine (6.23 g, 28 mmole), 3-(3-nitrophenoxy)-propyl bromide (7.28 g, 28 mmole) and diisopropylethylamine (24.4 ml, 0.14 mole) were combined in 200

ml of DMF and heated at 95 °C overnight under a nitrogen atmosphere. The solvent was then removed in vacuum and the residue dissolved in chloroform and washed with saturated aqueous sodium bicarbonate, dried over magnesium sulfate, filtered and concentrated in vacuum. The residue was column chromatographed on silica gel using 5% methanol/chloroform as eluant. The product-containing fractions were combined and concentrated in vacuum to give 5.86 g of a yellow solid. A portion (2.0 g, 4.9 mmole) of this solid was dissolved in ethanol (200 ml) and 1.0 g of 10% palladium on carbon added, along with 3.0 ml of 4 N isopropanolic HCl. The mixture was hydrogenated at 50 psi on a Parr apparatus overnight, filtered through celite, concentrated in vacuum to a white solid and recrystallized from methanol/isopropanol to give 2.06 g of title compound as an off-white solid, dihydrochloride, m.p. 220-222 °C.

| Elemental Analysis for: $C_{20}H_{26}N_2O_5$ • 2 HCl | | | |
|---|---|---|---|
| Calcd: | C, 53.70; | H, 6.31; | N, 6.26 |
| Found: | C, 53.61; | H, 6.49; | N, 6.18 |

2,3-Dihydro-6,7-ethylenedioxy-N-[3-(3-aminophenoxy)propyl]-1,4-benzodioxin-2-methanamine was produced in the same manner via the reaction of 2,3-dihydro-6,7-ethylenedioxy-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 231-235 °C) as described above.

| Elemental Analysis for: $C_{20}H_{24}N_2O_5$ • 2 HCl | | | |
|---|---|---|---|
| Calcd: | C, 53.94; | H, 5.88; | N, 6.29 |
| Found: | C, 53.96; | H, 6.15; | N, 5.97 |

**EXAMPLE 15**

**N-[3-(3-Aminophenoxy)propyl]-2,3-dihydro-5,6-dimethoxy-1,4-benzodioxin-2-methanamine**

2,3-Dihydro-5,6-dimethoxy-1,4-benzodioxin-2-methanamine (3.83 g, 17 mmole), 3-(3-nitrophenoxy)-propyl bromide (4.42 g, 17 mmole) and diisopropylethylamine (14.8 ml, 85 mmole) were combined in 200 ml of DMF and heated at 95 °C overnight under a nitrogen atmosphere. The solvent was then removed in vacuum and the residue dissolved in chloroform and washed with saturated aqueous sodium bicarbonate, dried over magnesium sulfate, filtered and concentrated in vacuum. The residue was column chromatographed on silica gel using 5% methanol/chloroform as eluant. The product-containing fractions were combined and concentrated in vacuum to give 3.53 g of a yellow oil. A portion (2.0 g, 4.9 mmole) of this solid was dissolved in ethanol (200 ml) and 1.0 g of 10% palladium on carbon added, along with 4 N isopropanolic HCl (to pH 3). The mixture was hydrogenated at 50 psi on a Parr apparatus overnight, filtered through celite, concentrated in vacuum to a white solid and recrystallized from methanol/isopropanol to give 1.77 g of title compound as a white solid, dihydrochloride, quarter hydrate, m.p. 205-207 °C.

| Elemental Analysis for: $C_{20}H_{26}N_2O_5$ • 2 HCl • 1/4 $H_2O$ | | | |
|---|---|---|---|
| Calcd: | C, 53.16; | H, 6.36; | N, 6.20 |
| Found: | C, 52.86; | H, 6.35; | N, 6.08 |

**EXAMPLE 16**

**N-[3-(3-Aminophenoxy)propyl]-2,3-dihydro-7-fluoro-1,4-benzodioxin-2-methanamine**

2,3-Dihydro-7-fluoro-1,4-benzodioxin-2-methanamine (4.5 g, 24 mmole), 3-(3-nitrophenoxy)propyl bromide (6.24 g, 24 mmole) and diisopropylethylamine (20.1 ml, 0.12 mole) were combined in 200 ml of DMF and heated at 95 °C overnight under a nitrogen atmosphere. The solvent was then removed in vacuum and the residue dissolved in chloroform and washed with saturated aqueous sodium bicarbonate, dried over

magnesium sulfate, filtered and concentrated in vacuum. The residue was column chromatographed on silica gel using 5% methanol/chloroform as eluant. The product-containing fractions were combined and concentrated in vacuum to give 3.0 g of a dark yellow oil. A portion (2.0 g, 5.5 mmole) of this was dissolved in ethanol (100 ml) and 1.0 g of 10% palladium on carbon added, along with 4 N isopropanolic HCl (to pH 3). The mixture was hydrogenated at 50 psi on a Parr apparatus overnight, filtered through celite, concentrated in vacuum to an off-white solid and column chromatographed on silica gel with 5% methanol/chloroform (with 1 ml/liter aqueous ammonia added). The product-containing fractions were combined and evaporated in vacuum and recrystallized from methanol/isopropanol with addition of 4 N HCl/isopropanol; however, the resulting solid proved to be contaminated with ammonium chloride. The product was reconverted to the free base by passing a methanol solution through Amberlite, IRA-400 (OH) ion exchange resin and concentration in vacuum. After overnight storage in vacuum, the residue was once again crystallized from methanol/isopropanol with addition of 4 N HCl/isopropanol to give 0.32 g of title compound as an tan solid, dihydrochloride, half hydrate, m.p. 209-212 °C.

| Elemental Analysis for: $C_{18}H_{21}FN_2O_3$ • 2 HCl • 1/2 $H_2O$ | | | |
|---|---|---|---|
| Calcd: | C, 52.18; | H, 5.84; | N, 6.76 |
| Found: | C, 52.09; | H, 5.62; | N, 6.56 |

(S)-2,3-Dihydro-7-fluoro-N-[3-(3-aminophenoxy)propyl]-1,4-benzodioxin-2-methanamine was produced in the same manner via the reaction of (S)-2,3-dihydro-7-fluoro-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 212-213 °C, $[\alpha]_D^{25}$ MeOH = -47.4) as described above.

| Elemental Analysis for: $C_{18}H_{21}FN_2O_3$ • 2 HCl | | | |
|---|---|---|---|
| Calcd: | C, 53.34; | H, 5.72; | N, 6.91 |
| Found: | C, 53.21; | H, 5.65; | N, 6.60 |

(R)-2,3-Dihydro-7-fluoro-N-[3-(3-aminophenoxy)propyl]-1,4-benzodioxin-2-methanamine was produced in the same manner via the reaction of (R)-2,3-dihydro-7-fluoro-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 222-224 °C, $[\alpha]_D^{25}$ MeOH = +47.9) as described above.

| Elemental Analysis for: $C_{18}H_{21}FN_2O_3$ • 2 HCl | | | |
|---|---|---|---|
| Calcd: | C, 53.34; | H, 5.72; | N, 6.91 |
| Found: | C, 53.57; | H, 5.82; | N, 6.72 |

## EXAMPLE 17

### (S)-N-[3-(3-Aminophenoxy)propyl]-2,3- dihydro-7-methoxy-1,4-benzodioxin-2-methanamine

(S)-2,3-Dihydro-7-methoxy-1,4-benzodioxin-2-methanamine hydrochloride (7.42 g, 32.0 mmole), 3-(3-nitrophenoxy)propyl bromide (7.644 g, 29.4 mmole) and diisopropylethylamine (8.0 ml, 46 mmole) were combined in 300 ml of DMF and heated at 86 °C for 26 hours under a nitrogen atmosphere. The solvent was then removed in vacuum and the residue dissolved in dichloromethane and washed with saturated aqueous sodium bicarbonate and with saturated aqueous sodium chloride, dried over magnesium sulfate, filtered and concentrated in vacuum. The residue was column chromatographed on 500 g of silica gel using 70% ethyl acetate/chloroform as eluant. The product-containing fractions were combined and concentrated in vacuum to give 4.5 g of a brown oil. This was dissolved in methanol (125 ml) and 0.86 g of 10% palladium on carbon added, along with 6.0 ml of 4 N isopropanolic HCl. The mixture was hydrogenated at 50 psi on a Parr apparatus overnight, filtered through celite, concentrated in vacuum and the residue crystallized from methanol/isopropanol with a further addition of 4N isopropanolic HCl to yield 2.07 g of the

title compound as a white solid, dihydrochloride, half hydrate, m.p. 204-208 °C.

| Elemental Analysis for: $C_{19}H_{24}N_2O_4$ • 2 HCl • 1/2 $H_2O$ | | | |
|---|---|---|---|
| Calcd: | C, 53.53; | H, 6.38; | N, 6.57 |
| Found: | C, 53.47; | H, 6.14; | N, 6.43 |

(R)-2,3-Dihydro-7-methoxy-N-[3-(3-aminophenoxy)propyl]-1,4-benzodioxin-2-methanamine was produced in the same manner via the reaction of (R)-2,3-dihydro-7-methoxy-1,4-benzodioxin-2-methanamine and 3-(3-nitrophenoxy)propyl bromide in DMF in the presence of diisopropylethylamine, followed by hydrogenation over 10% palladium on carbon and conversion to the dihydrochloride (m.p. 197-199 °C, $[\alpha]_D^{25}$ MeOH = +37.3) as described above.

| Elemental Analysis for: $C_{19}H_{24}N_2O_4$ • 2 HCl | | | |
|---|---|---|---|
| Calcd: | C, 54.68; | H, 6.28; | N, 6.71 |
| Found: | C, 54.50; | H, 6.37; | N, 6.68 |

## EXAMPLE 18

### N-[3-(3-Aminophenoxy)propyl]-2,3-dihydro-6,8-dimethoxy-1,4-benzodioxin-2-methanamine

2,3-Dihydro-6,8-dimethoxy-1,4-benzodioxin-2-methanamine hydrochloride (6.24 g, 23.8 mmole), 3-(3-nitrophenoxy)propyl bromide (6.51 g, 21.4 mmole) and diisopropylethylamine (4.5 ml, 26 mmole) were combined in 315 ml of DMF and heated at 80 - 100 °C for 24 hours under a nitrogen atmosphere. The solvent was then removed in vacuum and the residue dissolved in dichloromethane and washed with 300 ml of saturated aqueous sodium bicarbonate, dried over magnesium sulfate, filtered and concentrated in vacuum. The residue was column chromatographed on 300 g of silica gel using first 30% ethyl acetate/dichloromethane and then 1% methanol/dichloromethane as eluant. The product-containing fractions were combined and concentrated in vacuum to give 4.6 g of oil. This was dissolved in methanol (175 ml) and 0.7 g of 10% palladium on carbon added, along with 6.0 ml of 4 N isopropanolic HCl. The mixture was hydrogenated at 42 psi on a Part apparatus overnight, filtered through celite, concentrated in vacuum and the residue crystallized from methanol/isopropanol with a further addition of 4N isopropanolic HCl to yield 1.14 g of the title compound as a white solid, dihydrochloride, half hydrate, m.p. 123-128 °C.

| Elemental Analysis for: $C_{20}H_{26}N_2O_5$ • 2 HCl • 1/2 $H_2O$ | | | |
|---|---|---|---|
| Calcd: | C, 52.63; | H, 6.40; | N, 6.13 |
| Found: | C, 52.63; | H, 6.37; | N, 6.14 |

## EXAMPLE 19

### N-[3-(3-Aminophenoxy)propyl]-2,3-dihydro-7-hydroxy-8-methyl-1,4-benzodioxin-2-methanamine

2,3-Dihydro-7-hydroxy-8-methyl-1,4-benzodioxin-2-methanamine (0.70 g, 3.6 mmole), 3-(3-nitrophenoxy)propyl bromide (0.94 g, 3.6 mmole) and diisopropylethylamine (0.47 g, 3.6 mmole) were combined in 50 ml of DMF and heated at 80 °C for 24 hrs. under a nitrogen atmosphere. The solvent was then removed in vacuum and the residue dissolved in chloroform and washed with saturated aqueous sodium bicarbonate, dried over magnesium sulfate, filtered and concentrated in vacuum. The residue was column chromatographed on silica gel using first chloroform and then 2% methanol/chloroform as eluant. The product-containing fractions were combined and concentrated in vacuum to give a dark yellow oil. This was dissolved in methanol (100 ml) and 0.5 g of 10% palladium on carbon added, along with 5 ml of 4 N isopropanolic HCl. The mixture was hydrogenated at 50 psi on a Parr apparatus overnight, filtered through celite, concentrated in vacuum to an off-white solid and column chromatographed on silica gel with 5% methanol/ chloroform (with 1 ml/liter aqueous ammonia added). The product-containing fractions were

combined and evaporated in vacuum and recrystallized from isopropanol with addition of 4 N HCl/IPA to give 0.32 g of title compound as a yellow solid, dihydrochloride, hydrate, isopropanol solvate, m.p. 156 °C (d).

| Elemental Analysis for: $C_{19}H_{24}N_2O_4$ • 2 HCl • $H_2O$ • i-$C_3H_8O$ | | | |
|---|---|---|---|
| Calcd:<br>Found: | C, 54.21;<br>C, 54.62; | H, 7.45;<br>H, 7.07; | N, 5.75<br>N, 5.58 |

## EXAMPLE 20

### 7-[3-[[(2,3-Dihydro-1,4-benzodioxin-2-yl)methyl]amino]propoxy]-2H-1-benzopyran-2-one

2,3-Dihydro-1,4-benzodioxin-2-methanamine (1.7 g, 10 mmole), 7-(3-chloropropoxy)coumarin (2.4 g, 10 mmole), diisopropylethylamine (1.3 g, 10 mmole) and sodium iodide (5.0 g, 33 mmole) were combined in 100 ml of N-methylpyrrolidinone and heated at 80 °C for 24 hours under a nitrogen atmosphere. The solvent was then removed and replaced with 300 ml of dichloromethane. The mixture was washed with an equal volume of saturated aqueous sodium bicarbonate, with saturated aqueous sodium chloride, dried over sodium sulfate, filtered, and concentrated in vacuum. The residue was column chromatographed on 100 g of silica gel using chloroform as eluant. The product-containing fractions (Rf = 0.15 on silica gel tlc with 1% methanol/chloroform) were combined and concentrated in vacuum and the residue crystallized from isopropanol with the addition of 4 N isopropanolic HCl to give 1.1 g of title compound as a tan solid, monohydrochloride, m.p. 213-215 °C.

| Elemental Analysis for: $C_{21}H_{21}NO_5$ • HCl | | | |
|---|---|---|---|
| Calcd:<br>Found: | C, 62.45;<br>C, 62.67; | H, 5.49;<br>H, 5.65; | N, 3.47<br>N, 3.37 |

## EXAMPLE 21

### 7-[3-[[(2,3-Dihydro-5-methoxy-1,4-benzodioxin-2-yl)methyl]amino]propoxy]-2H-1-benzopyran-2-one

2,3-Dihydro-5-methoxy-1,4-benzodioxin-2-methanamine (1.29 g, 6.61 mmole), 7-(3-chloropropoxy)-coumarin (1.59 g, 6.7 mmole), diisopropylethylamine (2.5 ml, 14.35 mmole) and sodium iodide (4.96 g, 33.1 mmole) were combined in 75 ml of DMF and heated at 80 - 100 °C for 3 days under a nitrogen atmosphere. The solvent was then removed and replaced with dichloromethane. The mixture was washed with an equal volume of saturated aqueous sodium bicarbonate, with saturated aqueous sodium chloride, dried over magnesium sulfate, filtered, and concentrated in vacuum. The residue was column chromatographed on silica gel using 30% ethyl acetate/dichloromethane as eluant. The product-containing fractions (Rf = 0.25 on silica gel tlc with 2.5% methanol/dichloromethane) were combined and concentrated in vacuum and the residue (0.68 g) redissolved in dichloromethane and boiled on a hot plate, the dichloromethane gradually being replaced with isopropanol. 2 N HCl/isopropanol (4 ml) was added and the title compound was collected by filtration and dried in vacuum at 80 °C. The procedure yielded 0.64 g of white solid, monohydrochloride, m.p. 202-204 °C.

| Elemental Analysis for: $C_{22}H_{23}NO_6$ • HCl | | | |
|---|---|---|---|
| Calcd:<br>Found: | C, 60.91;<br>C, 61.04; | H, 5.57;<br>H, 5.46; | N, 3.23<br>N, 3.13 |

## EXAMPLE 22

### 7-[3-[[(2,3-Dihydro-7-phenylmethoxy-1,4-benzodioxin-2-yl)methyl]amino]propoxy]-2H-1-benzopyran-2-one

2,3-Dihydro-7-benzyloxy-1,4-benzodioxin-2-methanamine (1.47 g, 5.42 mmole), 7-(3-chloropropoxy)-coumarin (1.30 g, 5.45 mmole), diisopropylethylamine (4.0 ml, 23 mmole) and sodium iodide (4.17 g, 28 mmole) were combined in 150 ml of DMF and heated at 80 - 100 °C for 2 days under a nitrogen atmosphere. The solvent was then removed and replaced with dichloromethane. The mixture was washed with an equal volume of saturated aqueous sodium bicarbonate, with saturated aqueous sodium chloride, dried over magnesium sulfate, filtered, and concentrated in vacuum. The residue was column chromatographed on silica gel using first dichloromethane, then 0.5% methanol/dichloromethane, and finally 1% methanol/dichloromethane as eluant. The fractions containing material with Rf = 0.26 on silica gel tlc (2.5% methanol/chloroform) were combined and concentrated in vacuum and the residue crystallized from 50 ml of isopropanol with the addition of 4.0 ml of 4 N isopropanolic HCl to give 0.66 g of title compound as an off-white solid, monohydrochloride, m.p. 179-181 °C.

| Elemental Analysis for: $C_{28}H_{27}NO_6$ • HCl | | | |
|---|---|---|---|
| Calcd: | C, 65.94; | H, 5.53; | N, 2.75 |
| Found: | C, 65.60; | H, 5.19; | N, 2.91 |

## EXAMPLE 23

### 7-[3-[[(2,3-Dihydro-6-phenylmethoxy-1,4-benzodioxin-2-yl)methyl]amino]propoxy]-2H-1-benzopyran-2-one

2,3-Dihydro-6-benzyloxy-1,4-benzodioxin-2-methanamine (2.4 g, 8.85 mmole), 7-(3-chloropropoxy)-coumarin (2.15 g, 9.0 mmole), diisopropylethylamine (7.0 ml, 40.2 mmole) and sodium iodide (1.35 g, 9.0 mmole) were combined in 200 ml of DMF and heated at 108 °C for 2 days under a nitrogen atmosphere. The solvent was then removed and replaced with dichloromethane. The mixture was washed with an equal volume of water dried over magnesium sulfate, filtered, and concentrated in vacuum. The residue was column chromatographed on silica gel using 1% methanol/dichloromethane as eluant. The product-containing fractions (Rf = 0.27 on silica gel tlc with 2.5% methanol/dichloromethane) were combined and concentrated in vacuum and the residue redissolved in dichloromethane and boiled on a hot plate, the solvent gradually being replaced with isopropanol. Upon addition of 6 ml of 4N HCl/isopropanol and cooling, 1.22 g of the title compound, m.p. 184-187 °C, precipitated as a beige solid, monohydrochloride.

| Elemental Analysis for: $C_{28}H_{27}NO_6$ • HCl | | | |
|---|---|---|---|
| Calcd: | C, 65.94; | H, 5.53; | N, 2.75 |
| Found: | C, 65.74; | H, 5.49; | N, 2.96 |

## EXAMPLE 24

### 7-[3-[[(2,3-Dihydro-6-hydroxy-1,4-benzodioxin-2-yl)methyl]amino]propoxy]-2H-1-benzopyran-2-one

7-[3-[[(2,3-Dihydro-6-phenylmethoxy-1,4-benzodioxin-2-yl)methyl]amino]propoxy]-2H-1-benzopyran-2-one hydrochloride (0.82 g, 1.6 mmole), prepared in Example 23 above, was dissolved in a mixture of 150 ml of water and 10 ml of methanol, 300 mg of 5% palladium on carbon added, and the mixture hydrogenated at 25 psi on a Parr apparatus for 4.5 hours. It was then filtered through celite and concentrated to dryness in vacuum. The residue was redissolved in methanol and boiled on a hot plate, the methanol being gradually replaced with isopropanol, and an additional 2.8 ml of 4N HCl/isopropanol was added. Upon cooling 0.37 g of the title compound, m.p. 244 °C (d), precipitated as a white solid, monohydrochloride.

| Elemental Analysis for: $C_{21}H_{21}NO_6$ • HCl | | | |
|---|---|---|---|
| Calcd: | C, 60.07; | H, 5.28; | N, 3.34 |
| Found: | C, 59.77; | H, 5.38; | N, 3.11 |

**EXAMPLE 25**

**7-[3-[[(2,3-Dihydro-7-hydroxy-1,4-benzodioxin-2-yl)methyl]amino]propoxy]-2H-1-benzopyran-2-one**

2,3-Dihydro-7-hydroxy-1,4-benzodioxin-2-methanamine (3.00 g, 16.6 mmole), 7-(3-chloropropoxy)-coumarin (2.95 g, 12.4 mmole), diisopropylethylamine (8.0 ml, 46.0 mmole) and sodium iodide (2.58 g, 17.2 mmole) were combined in 150 ml of DMF and heated at 80 - 100 °C for 2 days under a nitrogen atmosphere. The solvent was then removed and replaced with dichloromethane. The mixture was washed with an equal volume of saturated aqueous sodium bicarbonate, with saturated aqueous sodium chloride, dried over magnesium sulfate, filtered, and concentrated in vacuum. The residue was column chromatographed on silica gel using first dichloromethane and then 0.25% methanol/dichloromethane as eluant. The product-containing fractions were combined and concentrated in vacuum and the pure free base (1.0 g, an additional 2.0 g were obtained of lower purity) redissolved in methanol and boiled on a hot plate, the methanol gradually being replaced with isopropanol. 4 N HCl/IPA was added and the title compound was collected by filtration and dried in vacuum at 80 °C. The procedure yielded 0.76 g of beige solid, monohydrochloride, m.p. 252 °C (d).

| Elemental Analysis for: $C_{21}H_{21}NO_6$ • HCl | | | |
|---|---|---|---|
| Calcd: | C, 60.07; | H, 5.28; | N, 3.34 |
| Found: | C, 59.73; | H, 5.59; | N, 3.31 |

**EXAMPLE 26**

**5-[3-[[(2,3-Dihydro-1,4-benzodioxin-2-yl)methyl]amino]propoxy]-2(1H)-quinolinone**

2,3-Dihydro-1,4-benzodioxin-2-methanamine (0.56 g, 4.0 mmole), 5-(3-chloropropoxy)carbostyril (0.70 g, 3.1 mmole), diisopropylethylamine (0.65 g, 5.0 mmole) and sodium iodide (1.0 g, 6.5 mmole) were combined in 50 ml of DMF and heated at 80 °C for 24 hours under a nitrogen atmosphere. The solvent was then removed and replaced with 250 ml of dichloromethane. The mixture was washed with an equal volume of saturated aqueous sodium bicarbonate, with saturated aqueous sodium chloride, dried over sodium sulfate, filtered, and concentrated in vacuum. The residue was column chromatographed on 50 g of silica gel with a gradient elution commencing with chloroform and ending with 2% methanol in chloroform. The product-containing fractions were combined and concentrated in vacuum and the residue crystallized from isopropanol with the addition of 4 N isopropanolic HCl to give 125 mg of title compound as a tan solid, monohydrochloride, quarter hydrate, m.p. 242-244 °C.

| Elemental Analysis for: $C_{21}H_{22}N_2O_4$ • HCl • 1/4 $H_2O$ | | | |
|---|---|---|---|
| Calcd: | C, 61.91; | H, 5.81; | N, 6.88 |
| Found: | C, 61.94; | H, 5.90; | N, 6.80 |

**EXAMPLE 27**

**(S)-7-[3-[[(2,3-Dihydro-6-hydroxy-1,4-benzodioxin-2-yl)methyl]amino]propoxy]-2H-1-benzopyran-2-one**

(S)-2,3-Dihydro-6-hydroxy-1,4-benzodioxin-2-methanamine (2.89 g, 16.0 mmole), 7-(3-chloropropoxy)-coumarin (3.46 g, 14.5 mmole), diisopropylethylamine (12.5 ml, 72 mmole) and sodium iodide (2.20 g, 14.7

mmole) were combined in 200 ml of DMF and heated at 80 - 100 °C for 24 hours under a nitrogen atmosphere. The solvent was then removed in vacuum. The residue was column chromatographed on silica gel using first 0.5% methanol/dichloromethane, then 1% methanol/dichloromethane, and finally 3% methanol/dichloromethane as eluant. The product-containing fractions (Rf = 0.45 on silica gel with 5% methanol/dichloromethane) were combined and rechromatographed on silica gel using first 75% ethyl acetate/dichloromethane and then 2% methanol/dichloromethane as eluant. The product-containing fractions were concentrated in vacuum and the pure free base (0.98 g) redissolved in methanol and boiled on a hot plate, the methanol gradually being replaced with isopropanol. 4 N HCl/isopropanol (7.0 ml) was added and the title compound was collected by filtration and dried in vacuum at 80 °C. The procedure yielded 0.71 g of white solid, monohydrochloride, m.p. 236-239 °C.

| Elemental Analysis for: $C_{21}H_{21}NO_6$ • HCl | | | |
|---|---|---|---|
| Calcd: | C, 60.07; | H, 5.28; | N, 3.34 |
| Found: | C, 59.95; | H, 5.26; | N, 3.53 |

**EXAMPLE 28**

**7-[3-[[(2,3-Dihydro-7-methoxy-1,4-benzodioxin-2-yl)methyl]amino]propoxy]-2H-1-benzopyran-2-one**

2,3-Dihydro-7-methoxy-1,4-benzodioxin-2-methanamine (3.8 g, 20 mmole), 7-(3-chloropropoxy)coumarin (4.8 g, 20 mmole), diisopropylethylamine (2.6 g 20 mmole) and sodium iodide (5.0 g, 33 mmole) were combined in 200 ml of DMF and heated at 100 °C for 24 hours under a nitrogen atmosphere. The solvent was then removed and replaced with 500 ml of dichloromethane. The mixture was washed with 300 ml portions of saturated aqueous sodium bicarbonate, with saturated aqueous sodium chloride, dried over sodium sulfate, filtered, and concentrated in vacuum. The residue was column chromatographed on 100 g of silica gel using first dichloromethane, then chloroform, and finally 2% methanol in chloroform as eluant. The product-containing tractions were combined and concentrated in vacuum and the residue crystallized from 100 ml of isopropanol with the addition of 5.0 ml of 4 N isopropanolic HCl to give 3.2 g of title compound as a white solid, monohydrochloride, m.p. 223-225 °C.

| Elemental Analysis for: $C_{22}H_{23}NO_6$ • HCl | | | |
|---|---|---|---|
| Calcd: | C, 60.90; | H, 5.58; | N, 3.23 |
| Found: | C, 61.09; | H, 5.42; | N, 3.32 |

**EXAMPLE 29**

**(R)-7-[3-[[(2,3-Dihydro-6-hydroxy-1,4-benzodioxin-2-yl)methyl]amino]propoxy]-2H-1-benzopyran-2-one**

(R)-2,3-Dihydro-6-hydroxy-1,4-benzodioxin-2-methanamine (2.58 g, 14.2 mmole), 7-(3-chloropropoxy)-coumarin (3.08 g, 12.9 mmole), diisopropylethylamine (10 ml, 57 mmole) and sodium iodide (1.98 g, 13.2 mmole) were combined in 175 ml of DMF and heated at 94 °C for 24 hours under a nitrogen atmosphere. The solvent was then removed in vacuum. The residue was twice column chromatographed on silica gel using 1% methanol/dichloromethane as eluant. The product-containing fractions (Rf = 0.45 on silica gel with 5% methanol/dichloromethane) were combined and concentrated in vacuum and the pure free base redissolved in methanol and boiled on a hot plate, the methanol gradually being replaced with isopropanol. 4 N HCL/isopropanol was added and the title compound was collected by filtration. After two additional recrystallizations from isopropanol, 0.35 g of white solid, monohydrochloride, m.p. 236-242 °C, was obtained.

| Elemental Analysis for: $C_{21}H_{21}NO_6 \cdot HCl$ | | | |
|---|---|---|---|
| Calcd: | C, 60.07; | H, 5.28; | N, 3.34 |
| Found: | C, 60.12; | H, 5.20; | N, 3.26 |

**EXAMPLE 30**

**7-[3-[[(2,3-Dihydro-6-acetoxy-1,4-benzodioxin-2-yl)methyl]amino]propoxy]-2H-1-benzopyran-2-one**

7-[3-[[(2,3-Dihydro-6-hydroxy-1,4-benzodioxin-2-yl)methyl]amino]propoxy]-2H-1-benzopyran-2-one hydrochloride (0.10 g 0.24 mmole), prepared as in example 24 above, was dissolved in 50 ml of glacial acetic acid and 3.05 ml (43 mmole) of acetyl chloride added in portions over a 30 minute period, during which the reaction mixture was gently warmed with a heat gun. The solvent and excess reagent were then removed in vacuum and diethyl ether added. Upon standing overnight, 0.030 g of the tide compound as a monohydrochloride, m.p. 175 °C, crystallized.

| Elemental Analysis for: $C_{23}H_{23}NO_7 \cdot HCl$ | | | |
|---|---|---|---|
| Calcd: | C, 59.81; | H, 5.24; | N, 3.03 |
| Found: | C, 59.42; | H, 5.39; | N, 2.98 |

**EXAMPLE 31**

**7-[3-[[(2,3-Dihydro-7-hydroxy-1,4-benzodioxin-2-yl)methyl]ethylamino]propoxy]-2H-1-benzopyran-2-one**

N-Ethyl-2,3-dihydro-7-hydroxy-1,4-benzodioxin-2-methanamine (2.11 g, 13.1 mmole), 7-(3-chloropropoxy)coumarin (3.44 g, 14.4 mmole), diisopropylethylamine (11 ml, 63.2 mmole) and sodium iodide (1.98 g, 13.2 mmole) were combined in 175 ml of DMF and heated at 94 °C for 2 days under a nitrogen atmosphere. The solvent was then removed in vacuum and the residue column chromatographed on silica gel using first 0.5% methanol/dichloromethane and then 1.5% methanol/dichloromethane as eluant. The product-containing fractions were combined and concentrated in vacuum and the residue redissolved in dichloromethane and boiled on a hot plate, the dichloromethane gradually being replaced with isopropanol. 4 N HCl/isopropanol (4 ml) was added and the title compound was collected by filtration. A second recrystallization from isopropanol yielded 0.73 g of white solid, monohydrochloride, m.p. 208-215 °C.

| Elemental Analysis for: $C_{23}H_{25}NO_6 \cdot HCl$ | | | |
|---|---|---|---|
| Calcd: | C, 61.68; | H, 5.85; | N, 3.13 |
| Found: | C, 61.71; | H, 5.97; | N, 2.96 |

**EXAMPLE 32**

**7-[3-[[(2,3-Dihydro-7-chloro-1,4-benzodioxin-2-yl)methyl]amino]propoxy]-2H-1-benzopyran-2-one**

2,3-Dihydro-7-chloro-1,4-benzodioxin-2-methanamine (1.72 g, 8.62 mmole), 7-(3-chloropropoxy)-coumarin (1.88 g, 7.88 mmole), diisopropylethylamine (7.5 ml, 43.1 mmole) and sodium iodide (1.37 g, 9.14 mmole) were combined in 200 ml of DMF and heated at 80 - 100 °C for 3 days under a nitrogen atmosphere. The solvent was then removed in vacuum and the residue was column chromatographed on silica gel using first 0.5% methanol/dichloromethane and then 1% methanol/dichloromethane as eluant. The product-containing fractions were combined and concentrated in vacuum, during which procedure the free base began to come out of solution. This material was collected by filtration and recrystallized from isopropanol with addition of 4 N HCl/isopropanol to give 0.27 g of white solid, monohydrochloride, m.p. 235-242 °C.

| Elemental Analysis for: $C_{21}H_{20}ClNO_5$ • HCl | | | |
|---|---|---|---|
| Calcd: | C, 57.55; | H, 4.83; | N, 3.20 |
| Found: | C, 57.19; | H, 4.86; | N, 3.07 |

## EXAMPLE 33

**7-[3-[[(2,3-Dihydro-7-methoxy-1,4-benzodioxin-2-yl)methyl]methylamino]propoxy]-2H-1-benzopyran-2-one**

7-[3-[[(2,3-Dihydro-7-methoxy-1,4-benzodioxin-2-yl)methyl]amino]propoxy]-2H-1-benzopyran-2-one hydrochloride (2.2 g, 5.0 mmole) was dissolved in 100 ml of DMF and 700 mg (5.0 mmole) of methyl iodide added, followed by 2.6 g (20 mmole) of diisopropylethylamine. The mixture was heated at 80 °C for 24 hours under a nitrogen atmosphere. The solvent was then removed in vacuum and replaced with 300 ml of dichloromethane. The mixture was washed with 300 ml portions of saturated aqueous sodium bicarbonate, saturated sodium chloride solution, dried over sodium sulfate, filtered and concentrated in vacuum. The residue was purified by column chromatography on 100 g of silica gel using chloroform as eluant and crystallized from isopropanol with addition of 2.5 ml of 4 N isopropanolic HCl to give 0.20 g of the title compound as a monohydrochloride, m.p. 176-178 °C.

| Elemental Analysis for: $C_{23}H_{25}NO_6$ • HCl | | | |
|---|---|---|---|
| Calcd: | C, 61.67; | H, 5.85; | N, 3.13 |
| Found: | C, 61.71; | H, 5.62; | N, 3.25 |

## EXAMPLE 34

**7-[3-[[(2,3-Dihydro-6-hydroxy-1,4-benzodioxin-2-yl)methyl]methylamino]propoxy]-2H-1-benzopyran-2-one**

N-Methyl-2,3-dihydro-6-hydroxy-1,4-benzodioxin-2-methanamine (3.68 g, 17.3 mmole), 7-(3-chloropropoxy)coumarin (4.15 g, 17.4 mmole), diisopropylethylamine (4.4 ml, 25.3 mmole) and sodium iodide (2.59 g, 17.3 mmole) were combined in 75 ml of DMF and heated at 96 °C for 26 hours under a nitrogen atmosphere. The solvent was then removed in vacuum and the residue was column chromatographed on silica gel using first 1% methanol/dichloromethane, then 2% methanol/dichloromethane as eluant. The product-containing fractions were combined and concentrated in vacuum and the residue crystallized from isopropanol with addition of 4 N HCl/isopropanol and diethyl ether to give 1.71 g of white solid, monohydrochloride, quarter hydrate, m.p. 186-193 °C.

| Elemental Analysis for: $C_{22}H_{23}NO_6$ • HCl • 1/4 $H_2O$ | | | |
|---|---|---|---|
| Calcd: | C, 60.28; | H, 5.63; | N, 3.19 |
| Found: | C, 60.21; | H, 5.59; | N, 3.17 |

## EXAMPLE 35

**(S)-7-[3-[[(2,3-Dihydro-6,7-methylenedioxy-1,4-benzodioxin-2-yl)methyl]amino]propoxy]-2H-1-benzopyran-2-one**

(S)-2,3-Dihydro-6,7-methylenedioxy-1,4-benzodioxin-2-methanamine hydrochloride (5.50 g, 22.4 mmole), 7-(3-chloropropoxy)coumarin (5.49 g, 23.0 mmole) diisopropylethylamine (39.0 ml, 224 mmole) and sodium iodide (3.45 g, 23.0 mmole) were combined in 200 ml of DMF and heated at 80 °C for 24 hours under a nitrogen atmosphere. The solvent was then removed in vacuum and replaced with chloroform. The mixture was treated with an equal volume of saturated aqueous sodium bicarbonate and the aqueous phase

was then back-extracted with 3:1 chloroform-isopropanol. The combined organic extracts were washed with water, dried over anhydrous magnesium sulfate, filtered and concentrated under vacuum. The residue was chromatographed on a silica gel column using dichloromethane as eluant. The product-containing fractions (Rf = 0.59 on silica gel tlc with 1:5:95 ammonium hydroxide-methanol-chloroform) were combined and concentrated in vacuum, and the residue redissolved in methanol and acidified with 4N HCl/isopropanol to pH < 3 in an ice bath to precipitate a white solid. This was recrystallized from methanol and triturated with a small amount of isopropanol to give 1.50 g of the title compound as an off-white crystalline, solid, monohydrochloride, m.p. 244-245 °C.

| Elemental Analysis for: $C_{22}H_{21}NO_7$ • HCl | | | |
|---|---|---|---|
| Calcd: | C, 59.00; | H, 4.95; | N, 3.13 |
| Found: | C, 59.07; | H, 4.92; | N, 2.94 |

Racemic 7-[3-[[(2,3-dihydro-6,7-methylenedioxy-1,4-benzodioxin-2-yl)methyl]amino]propoxy]-2H-1-benzopyran-2-one was produced in the same manner via the reaction of racemic 2,3-dihydro-6,7-methylenedioxy-1,4-benzodioxin-2-methanamine with 7-(3-bromopropoxy)coumarin in DMF in the presence of diisopropylethylamine followed by conversion to the hydrochloride salt (m.p. 220-223 °C) as described above.

| Elemental Analysis for: $C_{22}H_{21}NO_7$ • HCl | | | |
|---|---|---|---|
| Calcd: | C, 58.99; | H, 4.95; | N, 3.13 |
| Found: | C, 58.59; | H, 4.98; | N, 3.13 |

**EXAMPLE 36**

**(S)-7-[3-[[(2,3-Dihydro-6,7-methylenedioxy-1,4-benzodioxin-2-yl)methyl]ethylamino]propoxy]-2H-1-benzopyran-2-one**

(S)-7-[3-[[(2,3-Dihydro-6,7-methylenedioxy-1,4-benzodioxin-2-yl)methyl]amino]propoxy]-2H-1-benzopyran-2-one hydrochloride (0.60 g, 1.3 mmole), iodoethane (0.16 ml, 2.0 mmole) and diisopropylethylamine (2.26 ml, 13.0 mmole) were combined in 50 ml of DMF and heated at 60 °C for 2 days under a nitrogen atmosphere. The solvent was then removed in vacuum and the residue was dissolved in dichloromethane and washed with an equal volume of aqueous saturated sodium bicarbonate. The aqueous portion was back-extracted with dichloromethane and the combined organic portions were washed with water, dried over anhydrous magnesium sulfate, filtered, and concentrated under reduced pressure. The residue was chromatographed on a silica gel column using dichloromethane as eluant. The product-containing fractions were combined and concentrated in vacuum, and the residue redissolved in methanol and acidified with 4N HCl/isopropanol to pH < 3 in an ice bath. The mixture was diluted with isopropanol to precipitate a white solid, which was recrystallized from methanol to give 0.25 g of the title compound as the monohydrochloride, m.p. 209 °C.

| Elemental Analysis for: $C_{24}H_{25}NO_7$ • HCl | | | |
|---|---|---|---|
| Calcd: | C, 60.57; | H, 5.51; | N, 2.94 |
| Found: | C, 60.44; | H, 5.27; | N, 2.91 |

**EXAMPLE 37**

**(S)-7-[3-[[(2,3-Dihydro-7-methoxy-1,4-benzodioxin-2-yl)methyl]amino]propoxy]-2H-1-benzopyran-2-one**

(S)-2,3-Dihydro-7-methoxy-1,4-benzodioxin-2-methanamine hydrochloride (11.5 g, 49.8 mmole), 7-(3-bromopropoxy)coumarin (14.2 g, 50.0 mmole), and diisopropylethylamine (12 ml, 68.9 mmole) were

combined in 350 ml of DMF and heated at 90 °C for 24 hours uunder a nitrogen atmosphere. The solvent was then removed in vacuum and replaced with 300 ml of dichloromethane. Upon addition of an equal volume of saturated aqueous sodium bicarbonate, a white solid precipitated. This was filtered and redissolved in 550 ml of warm methanol and 35 ml of 4N isopropanolic HCl was added. The solution was boiled on a hot plate and the solvent gradually replaced with isopropanol. Upon cooling, 0.54 g of the title compound precipitated as a white solid, monohydrochloride, quarter hydrate, m.p. 225-227°C. Subsequent crops yielded as additional 6.1 g of product.

| Elemental Analysis for: $C_{22}H_{23}NO_6$ • HCl • 1/4 $H_2O$ | | | |
|---|---|---|---|
| Calcd: | C, 60.27; | H, 5.57; | N, 3.19 |
| Found: | C, 59.98; | H, 5.45; | N, 3.04 |

(S)-7-[3-[[(2,3-dihydro-7-fluoro-1,4-benzodioxin-2-yl)methyl]amino]propoxy]-2H-1-benzopyran-2-one was produced in the same manner via the reaction of (S)-2,3-dihydro-7-fluoro-1,4-benzodioxin-2-methanamine with 7-(3-bromopropoxy)coumarin in DMF in the presence of diisopropylethylamine followed by conversion to the hydrochloride salt (m.p. 224°C, $[\alpha]_D^{25}$ MeOH = -46.5) as described above.

| Elemental Analysis for: $C_{21}H_{20}FNO_5$ • HCl | | | |
|---|---|---|---|
| Calcd: | C, 59.79; | H, 5.02; | N, 3.32 |
| Found: | C, 60.15; | H, 5.02; | N, 3.31 |

## EXAMPLE 38

**7-[3-[[(2,3-Dihydro-6,8-dimethoxy-1,4-benzodioxin-2-yl)methyl]amino]propoxy]-2H-1-benzopyran-2-one**

2,3-Dihydro-6,8-dimethoxy-1,4-benzodioxin-2-methanamine hydrochloride (3.58 g, 13.7 mmole), 7-(3-bromopropoxy)coumarin (3.35 g, 11.8 mmole), and diisopropylethylamine (3.25 ml, 18.7 mmole) were combined in 200 ml of DMF and heated at 96 °C for 2 days under a nitrogen atmosphere. The solvent was then removed and replaced with dichloromethane. The mixture was washed with an equal volume of saturated aqueous sodium bicarbonate, dried over magnesium sulfate, filtered and concentrated in vacuum. The residue was column chromatographed on 300 g of silica gel using first dichloromethane and then 1% methanol/dichlormethane as eluant. The product-containing fractions were combined and concentrated in vacuum and the free base thus obtained redissolved in methanol and 7 ml of 4N isopropanolic HCl added. The mixture was brought to a boil on a hot plate and the solvent gradually replaced with isopropanol. Upon cooling, 0.87 g of the title compound (m.p. 184-187°C) precipitated as a white solid, monohydrochloride.

| Elemental Analysis for: $C_{23}H_{25}NO_7$ • HCl | | | |
|---|---|---|---|
| Calcd: | C, 59.55; | H, 5.65; | N, 3.02 |
| Found: | C, 59.22; | H, 5.63; | N, 3.08 |

## EXAMPLE 39

**(S)-7-[3-[[(2,3-Dihydro-7-hydroxy-1,4-benzodioxin-2-yl)methyl]amino]propoxy]-2H-1-benzopyran-2-one**

(S)-2,3-Dihydro-7-hydroxy-1,4-benzodioxin-2-methanamine (3.53 g, 16.2 mmole), 7-(3-bromopropoxy)-coumarin (4.17 g, 14.7 mmole), and diisopropylethylamine (5.00 ml, 28.7 mmole) were combined in 300 ml of DMF and heated at 86 °C for 24 hours under a nitrogen atmosphere. The solvent was then removed in vacuum and replaced with dichloromethane. The mixture was washed with an equal volume of saturated aqueous sodium bicarbonate, dried over magnesium sulfate, filtered and concentrated in vacuum. The residue was column chromatographed on 300 g of silica gel using first 75% ethyl acetate/dichloromethane

and then 2% methanol/dichloromethane as eluant. The product-containing fractions were concentrated in vacuum and the free base redissolved in warm methanol. Five ml of 4N isopropanolic HCl was added, the mixture was brought to a boil on a hot plate and the solvent gradually replaced with isopropanol. Upon cooling, 2.24 g of the title compound precipitated as an white solid, monohydrochloride, m.p. 245-248 ° C.

| Elemental Analysis for: $C_{21}H_{21}NO_6$ • HCl | | | |
|---|---|---|---|
| Calcd: | C, 60.07; | H, 5.28; | N, 3.34 |
| Found: | C, 59.92; | H, 5.31; | N, 3.31 |

**EXAMPLE 40**

**(S)-7-[3-[[(2,3-Dihydro-7-hydroxy-1,4-benzodioxin-2-yl)methyl]ethylamino]propoxy]-2H-1-benzopyran-2-one**

(S)-7-[3-[[(2,3-Dihydro-7-hydroxy-1,4-benzodioxin-2-yl)methyl]amino]propoxy]-2H-1-benzopyran-2-one hydrochloride (0.89 g, 2.13 mmole), diisopropylethylamine (0.80 ml, 4.59 mmole) and iodoethane (0.24 ml, 3.0 mmole) were combined in DMF and heated at 60 ° C for 15 hours under a nitrogen atmosphere. The solvent was then removed in vacuum and replaced with 75 ml ofdichloromethane. The mixture was washed with an equal volume of saturated aqueous sodium bicarbonate, dried over magnesium sulfate, filtered and concentrated in vacuum. The residue (1.82 g) was column chromatographed on 75 g of silica gel using first 0.5% methanol/dichloromethane and then 2% methanol/dichloromethane as eluant. The product-containing fractions were concentrated in vacuum and the free base redissolved in methanol. 2.8 ml of 4N isopropanolic HCl was added, the mixture was brought to a boil on a hot plate and the solvent gradually replaced with isopropanol. Upon cooling, 0.66 g of the title compound precipitated as a yellow solid, monohydrochloride, quarter hydrate, m.p. 204-207 ° C.

| Elemental Analysis for: $C_{23}H_{25}NO_6$ • HCl • 1/4 $H_2O$ | | | |
|---|---|---|---|
| Calcd: | C, 61.06; | H, 5.90; | N, 3.09 |
| Found: | C, 61.22; | H, 5.75; | N, 2.93 |

**EXAMPLE 41**

**7-[3-[[(2,3-Dihydro-1,4-benzodioxin-2-yl)methyl]ethylamino]propoxy]-2H-1-benzopyran-2-one**

N-Ethyl-2,3-dihydro-1,4-benzodioxin-2-methanamine (4.67 g, 24.2 mmole), 7-(3-bromopropoxy)coumarin (6.92 g, 24.4 mmole), and diisopropylethylamine (6.70 ml, 38.5 mmole) were combined in 300 ml of DMF and heated at 76 ° C for 24 hours under a nitrogen atmosphere. The solvent was then removed in vacuum and replaced with dichloromethane. The mixture was washed with an equal volume of saturated aqueous sodium bicarbonate, dried over magnesium sulfate, filtered and concentrated in vacuum. The residue (10 g) was column chromatographed on 225 g of silica gel using 40% ethyl acetate/dichloromethane as eluant. The product-containing fractions were concentrated in vacuum and the residue crystallized from isopropanol with the addition of 30 ml of 4N isopropanolic HCl to give 4.94 g of the title compound as a white solid, monohydrochloride, m.p. 147-152 ° C.

| Elemental Analysis for: $C_{23}H_{25}NO_5$ • HCl | | | |
|---|---|---|---|
| Calcd: | C, 63.95; | H, 6.07; | N, 3.24 |
| Found: | C, 63.74; | H, 5.91; | N, 3.06 |

The compounds of the invention may be represented by the formula

$Y^1$-$CH_2$-$NR^3$-$CH_2$-$Y^2$

and the pharmaceutically acceptable salts thereof where R3 is hydrogen or alkyl of 1 to 6 carbon atoms; one of $Y^1$ and $Y^2$ is a group having the formula

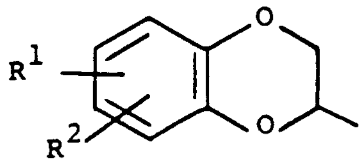

where $R^1$ and $R^2$ are as defined above and the other of $Y^1$ and $Y^2$ is a group having the formula

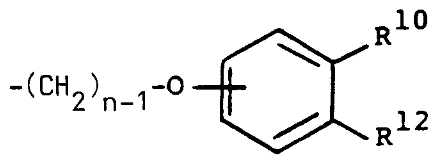

where $R^{10}$, $R^{12}$, n and the dotted line are as defined above. The compounds of the invention may be prepared by a process in which

(a)

an amine having the formula

$Y^1$-$CH_2$-$NHR^3$

is N-alkylated to introduce a substituted alkyl group having the formula -$CH_2$-$Y^2$ where $Y^1$, $Y^2$ and $R^3$ are as defined above except that an amino or mono- alkylamino group represented by $R^1$, $R^2$ or -$NR^4R^5$ or a hydroxy group represented by $R^1$ or $R^2$ may be temporarily protected by a removable protecting group; or

(b)

an amine having the formula

$Y^1$-$CH_2$-$NHR^3$

is reductively N-alkylated with an aldehyde having the formula $Y^2$-CHO where $Y^1$, $Y^2$ and $R^3$ are as defined above; or

(c)

an amine having the formula

$Y^1$-CO-$NR^3$-$CH_2$-$Y^2$

where $Y^1$, $Y^2$ and $R^3$ are as defined above, is reduced to convert the carbonyl group thereof to methylene; or

(d)

a nitro compound having the formula

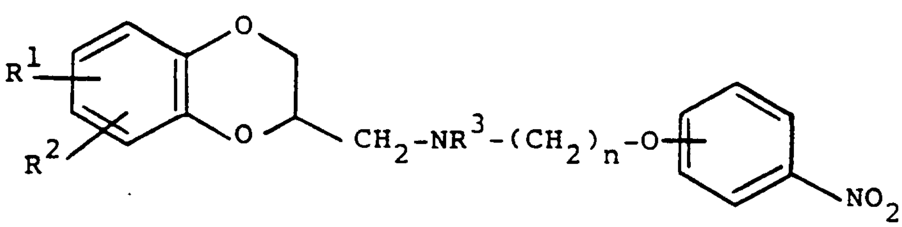

where $R^1$, $R^2$, $R^3$ and n are as defined above, is reduced to convert the nitro group to amino; or
(e)
an amine having the formula

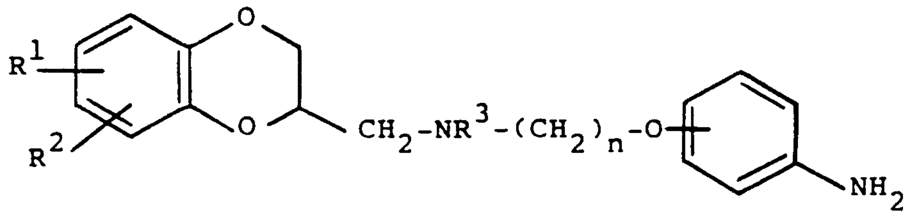

is subjected to alkylation, acylation, or alkyl- or arylsulfonylation to convert $-NH_2$ into another meaning of $-NR^4R^5$; and, where appropriate, a protecting group is removed and, if desired, a compound of the invention is converted into a pharmaceutically acceptable salt thereof or, if desired, a salt of a compound of the invention is converted into a compound of the invention.

Pharmaceutical preparations may be prepared according to the invention by bringing an active compound into combination or association with a pharmaceutical carrier.

## Claims

**Claims for the following Contracting States : AT, BE, CH, DE, DK, FR, GB, IT, LI, LU, NL, PT, SE**

**1.** A compound of the formula:

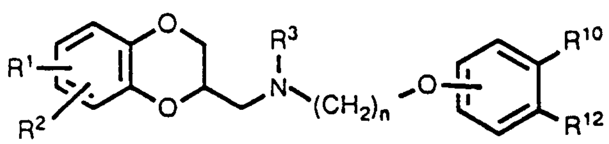

wherein

| | |
|---|---|
| $R^1$ and $R^2$ | are, independently, hydrogen, alkyl of 1 to 6 carbon atoms, alkoxy of 1 to 6 carbon atoms, aralkoxy of 7 to 12 carbon atoms, alkanoyloxy of 2 to 6 carbon acorns, hydroxy, halo, amino, mono- or dialkylamino in which each alkyl group contains from 1 to 6 carbon atoms, alkanamido of 2 to 6 carbon atoms, or sulfonamido, or $R^1$ and $R^2$ together form methylenedioxy, ethylenedioxy, or propylenedioxy; |
| $R^3$ | is hydrogen or alkyl of 1 to 6 carbon atoms; |
| n | is one of the integers 2, 3 or 4; |
| $R^{12}$ | is $NR^4R^5$ in which $R^4$ and $R^5$ are, independently, hydrogen, alkyl of 1 to 6 carbon atoms, cycloalkyl of 4 to 7 carbon atoms, alkanoyl of 2 to 6 carbon atoms, aroyl of 7 to 12 carbon atoms, alkylsulfonyl of 1 to 6 carbon atoms or arylsulfonyl of 6 to 10 carbon atoms, or $R^4$ and $R^5$ together form a 3-7 membered polymethylene ring; |
| $R^{10}$ | is hydrogen or, when taken with $R^{12}$, $R^{10}$ and $R^{12}$ complete the six membered ring - |

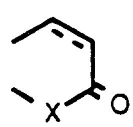

in which X is O or $NR^8$, in which $R^8$ is hydrogen or alkyl of 1 to 6 carbon atoms;
or a pharmaceutically acceptable salt thereof.

**2.** A compound of Claim 1 in which $R^1$ and $R^2$ are, independently, hydrogen, fluoro, hydroxy, alkoxy of 1 to 6 carbon atoms or alkanoyloxy of 2 to 6 carbon atoms, or together form a methylenedioxy, ethylenedioxy or propylenedioxy ring; $R^4$ and $R^5$ are, independently, hydrogen or alkyl of 1 to 6 carbon atoms; and the bond between oxygen and the anilino moiety is in meta position, or the $-(CH_2)_n-O-$ link is to the 7-position of the coumarin or carbostyril nucleus, or a pharmaceutically acceptable salt thereof.

**3.** A compound of Claim 1 of the formula:

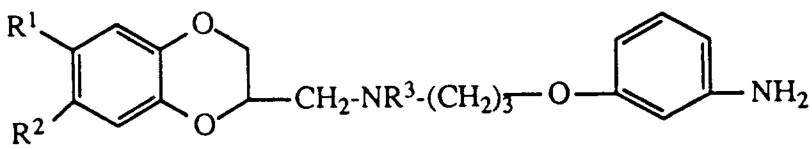

in which
$R^1$ and $R^2$ are, independently, hydrogen, fluoro, hydroxy, alkoxy of 1 to 4 carbon atoms, alkanoyloxy of 2 to 4 carbon atoms or together form an alkylenedioxy ring of 1 to 3 carbon atoms;
$R^3$ is hydrogen or alkyl of 1 to 4 carbon atoms;
or a pharmaceutically acceptable salt thereof, where the benzodioxan methanamine moiety is in the S-configuration.

**4.** A compound of Claim 1 of the formula:

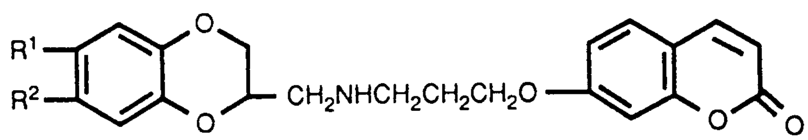

in which $R^1$ and $R^2$ are, independently, hydrogen, fluoro, hydroxy, alkoxy of 1 to 6 carbon atoms, alkanoyloxy of 2 to 6 carbon atoms, or, taken together, $R^1$ and $R^2$ are methylenedioxy, ethylenedioxy or propylenedioxy, or a pharmaceutically acceptable salt thereof, wherein the benzodioxan methamine moiety is in the S-configuration.

**5.** A compound according to claim 1, which is selected from
N-[3-(3-aminophenoxy)propyl]-2,3-dihydro-7-hydroxy-8-methyl-1,4-benzodioxin-2-methanamine;
N-[3-(3-aminophenoxy)propyl]-2,3-dihydro-6,8-dimethoxy-1,4-benzodioxin-2-methanamine; N-[3-(3-aminophenoxy)propyl]-2,3-dihydro-7-fluoro-1,4-benzodioxin-2-methanamine;
N-[3-(3-aminophenoxy)propyl]-2,3-dihydro-5,6-dimethoxy-1,4-benzodioxin-2-methanamine;
N-[3-(3-aminophenoxy)propyl]-2,3-dihydro-6,7-ethylenedioxy-1,4-benzodioxin-2-methanamine; N-[3-(3-aminophenoxy)propyl]-2,3-dihydro-6,7-dimethoxy-1,4-benzodioxin-2-methanamine; N-[3-(3-aminophenoxy)propyl]-2,3-dihydro-7-chloro-1,4-benzodioxin-2-methanamine;
N-[3-(3-aminophenoxy)propyl]-2,3-dihydro-8-hydroxy-1,4-benzodioxin-2-methanamine;
N-[3-(3-aminophenoxy)propyl]-2,3-dihydro-8-methoxy-1,4-benzodioxin-2-methanamine;
N-[3-(3-aminophenoxy)propyl]-2,3-dihydro-6,7-methylenedioxy-1,4-benzodloxin-2-methanamine; N-[3-(3-aminophenoxy)propyl]-2,3-dihydro-7-methoxy-1,4-benzodioxin-2-methanamine;
N-[3-(3-aminophenoxy)propyl]-2,3-dihydro-6-methyl-7-methoxy-1,4-benzodioxin-2-methanamine;
N-[3-(3-aminophenoxy)propyl]-2,3-dihydro-5-hydroxy-1,4-benzodioxin-2-methanamine;
N-[3-(3-aminophenoxy)propyl]-2,3-dihydro-6-hydroxy-1,4-benzodioxin-2-methanamine;
N-[3-(3-aminophenoxy)propyl]-2,3-dihydro-7-hydroxy-1,4-benzodioxin-2-methanamine;
N-[3-(3-aminophenoxy)propyl]-2,3-dihydro-6-methyl-7-hydroxy-1,4-benzodioxin-2-methanamine;
N-[3-(3-aminophenoxy)propyl]-2,3-dihydro-5-methoxy-1,4-benzodioxin-2-methanamine;
and the pharmaceutically acceptable salts thereof.

**6.** A compound according to claim 1, which is selected from
(R)-N-[3-(3-aminophenoxy)propyl]-2,3-dihydro-7-hydroxy-1,4-benzodioxin-2-methanamine;
(R)-N-[3-(3-aminophenoxy)propyl]-2,3-dihydro-6,7-methylenedioxy-1,4-benzodioxin-2-methanamine;
(R)-N-[3-(3-aminophenoxy)propyl]-2,3-dihydro-7-fluoro-1,4-benzodioxin-2-methanamine;
(R)-N-[3-(3-aminophenoxy)propyl]-2,3-dihydro-7-methoxy-1,4-benzodioxin-2-methanamine;
and the pharmaceutically acceptable salts thereof.

**7.** A compound as claimed in claim 1, which is selected from
(S)-N-[3-(3-aminophenoxy)propyl]-2,3-dihydro-7-hydroxy-1,4-benzodioxin-2-methanamine;
(S)-N-[3-(3-aminophenoxy)propyl]-2,3-dihydro-6,7-methylenedioxy-1,4-benzodioxin-2-methanamine;
(S)-N-[3-(3-aminophenoxy)propyl]-2,3-dihydro-7-fluoro-1,4-benzodioxin-2-methanamine;
(S)-N-[3-(3-aminophenoxy)propyl]-2,3-dihydro-7-methoxy-1,4-benzodioxin-2-methanamine;
and the pharmaceutically acceptable salts thereof.

**8.** A compound as claimed in claim 1, which is selected from
7-[3-[[(2,3-dihydro-1,4-benzodioxin-2-yl)methyl]amino] propoxy]-2H-1-benzopyran-2-one;
7-[3-[[(2,3-dihydro-6,8-methoxy-1,4-benzodioxin-2-yl) methyl]amino]propoxy]-2H-1-benzopyran-2-one;
7-[3-[[(2,3-dihydro-5-methoxy-1,4-benzodioxin-2-yl) methyl]amino]propoxy]-2H-1-benzopyran-2-one;
7-[3-[[(2,3-dihydro-7-chloro-1,4-benzodioxin-2-yl) methyl]amino]propoxy]-2H-1-benzopyran-2-one;
7-[3-[[(2,3-dihydro-7-phenylmethoxy-1,4-benzodioxin-2-yl)methyl]amino]propoxy]-2H-1-benzopyran-2-one;
7-[3-[[(2,3-dihydro-6-phenylmethoxy-1,4-benzodioxin-2-yl)methyl]amino]propoxy]-2H-1-benzopyran-2-one;
7-[3-[[(2,3-dihydro-6-hydroxy-1,4-benzodioxin-2-yl)methyl]amino]propoxy]-2H-1-benzopyran-2-one;
7-[3-[[(2,3-dihydro-7-hydroxy-1,4-benzodioxin-2-yl)methyl]amino]propoxy]-2H-1-benzopyran-2-one;
7-[3-[[(2,3-dihydro-7-methoxy-1,4-benzodioxin-2-yl)methyl]amino]propoxy]-2H-1-benzopyran-2-one;
7-[3-[[(2,3-dihydro-6-acetoxy-1,4-benzodioxin-2-yl)methyl]amino]propoxy]-2H-1-benzopyran-2-one;
7-[3-[[(2,3-dihydro-7-hydroxy-1,4-benzodioxin-2-yl) methyl]ethylamino]propoxy]-2H-1-benzopyran-2-one;
7-[3-[[(2,3-dihydro-1,4-benzodioxin-2-yl) methyl]ethylamino]propoxy]-2H-1-benzopyran-2-one;
7-[3-[[(2,3-dihydro-7-methoxy-1,4-benzodioxin-2-yl) methyl]methylamino]propoxy]-2H-1-benzopyran-2-one;
7-[3-[[(2,3-dihydro-6-hydroxy-1,4-benzodioxin-2-yl) methyl]methylamino]propoxy]-2H-1-benzopyran-2-one;
and the pharmaceutically acceptable salts thereof.

**9.** A compound as claimed in claim 1, which is selected from
(S)-7-[3-[[(2,3-dihydro-7-hydroxy-1,4-benzodioxin-2-yl)methyl]ethylamino]propoxy]-2H-1-benzopyran-2-one;
(S)-7-[3-[[(2,3-dihydro-7-hydroxy-1,4-benzodioxin-2-yl)methyl]amino]propoxy]-2H-1-benzopyran-2-one;
(S)-7-[3-[[(2,3-dihydro-7-methoxy-1,4-benzodioxin-2-yl)methyl]amino]propoxy]-2H-1-benzopyran-2-one;
(S)-7-[3-[[(2,3-dihydro-6,7-methylenedioxy-1,4-benzodioxin-2-yl)methyl]ethylamino]propoxy]-2H-1- benzopyran-2-one;
(S)-7-[3-[[(2,3-dihydro-6,7-methylenedioxy-1,4-benzodioxin-2-yl)methyl]amino]propoxy]-2H-1- benzopyran-2-one;
(S)-7-[3-[[(2,3-dihydro-6-hydroxy-1,4-benzodioxin-2-yl)methyl]amino]propoxy]-2H-1-benzopyran-2-one;
and their pharmaceutically acceptable salts.

**10.** A compound as claimed in claim 1, which is selected from
5-[3[[(2,3-dihydro-1,4-benzodioxin-2-yl)methyl]amino] propoxy]-2(1H)-quinoline;
(R)-7-[3-[[(2,3-dihydro-6-hydroxy-1,4-benzodioxin-2-yl) methyl)amino]propoxy]-2H-1-benzopyran-2-one
and their pharmaceutically acceptable salts.

**11.** A pharmaceutical composition comprising a compound as claimed in any one of claims 1 to 10 in combination or association with a pharmaceutical carrier.

**Claims for the following Contracting States : ES, GR**

**1.** A process for the preparation of a compound having the formula:

$Y^1$-$CH_2$-$NR^3$-$CH_2$-$Y^2$

or a pharmaceutically acceptable salt thereof where R3 is hydrogen or alkyl of 1 to 6 carbon atoms; $Y^1$ and $Y^2$ is a group having the formula

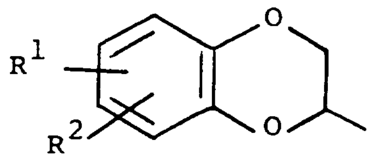

where $R^1$ and $R^2$ are independently, hydrogen, alkyl of 1 to 6 carbon atoms, alkoxy of 1 to 6 carbon atoms, aralkoxy of 7 to 12 carbon atoms, alkanoyloxy of 2 to 6 carbon atoms, hydroxy, halo, amino, mono- or dialkylamino in which each alkyl group contains from 1 to 6 carbon atoms, alkanamido of 2 to 6 carbon atoms, sulfonamido, or $R^1$ and $R^2$ together form methylenedioxy, ethylenedioxy, or propylenedioxy; and the other of $Y^1$ and $Y^2$ is a group having the formula

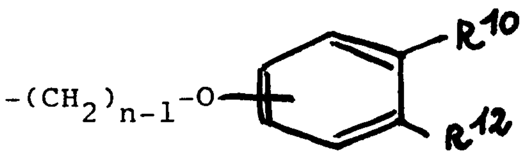

n is one of the integers 2, 3 or 4;
$R^{12}$ is $NR^4R^5$ in which $R^4$ and $R^5$ are independently hydrogen, alkyl of of 1 to 6 carbon atoms, cycloalkyl of 4 to 7 carbon atoms, alkanoyl of 2 to 6 carbon atoms, aroyl of 7 to 12 carbon atoms, alkylsulfonyl of 1 to 6 carbon atoms or arylsulfonyl of 6 to 10 carbon atoms, or $R^4$ and $R^5$ together form a 3-7 membered polymethylene ring;
$R^{10}$ is hydrogen or, when taken with $R^{12}$, $R^{10}$ and $R^{12}$ complete the six membered ring

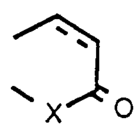

in which the dotted line means optional unsaturation and X is O or $NR^8$, in which $R^8$ is hydrogen or alkyl of 1 to 6 carbon atoms; in which

(a) an amine having the formula

$Y^1$-$CH_2$-$NHR^3$

is N-alkylated to introduce a substituted alkyl group having the formula -$CH_2$-$Y^2$ where $Y^1$, $Y^2$ and $R^3$ are as defined above except that an amino or mono- alkylamino group represented by $R^1$, $R^2$ or -$NR^4R^5$ or a hydroxy group represented by $R^1$ or $R^2$ may be temporarily protected by a removable protecting group; or

(b) an amine having the formula

$Y^1$-$CH_2$-$NHR^3$

is reductively N-alkylated with an aldehyde having the formula $Y^2$-CHO where $Y^1$, $Y^2$ and $R^3$ are as defined above; or

(c) an amine having the formula

$Y^1$-CO-$NR^3$-$CH_2$-$Y^2$

where $Y^1$, $Y^2$ and $R^3$ are as defined above, is reduced to convert the carbonyl group thereof to methylene; or

(d) a nitro compound having the formula

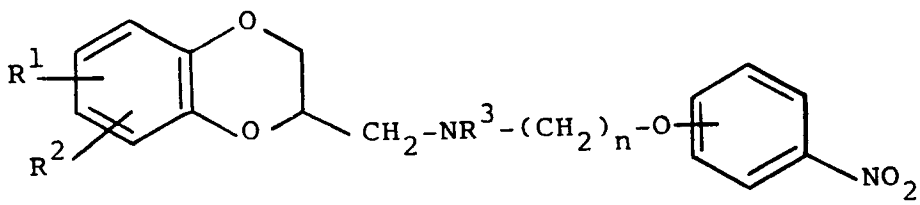

where $R^1$, $R^2$, $R^3$ and n are as defined above, is reduced to convert the nitro group to amino; or

(e) an amine having the formula

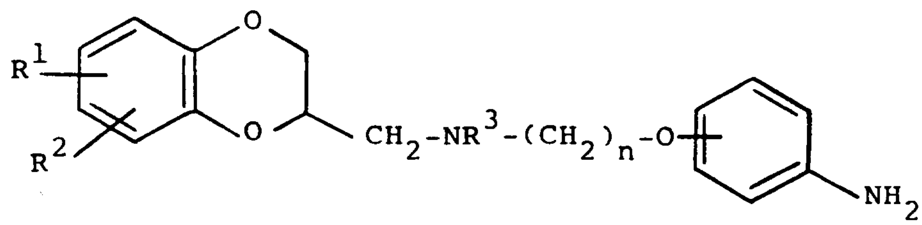

is subjected to alkylation, acylation, or alkyl- or arylsulfonylation to convert $-NH_2$ into another meaning of $-NR^4R^5$; and, where appropriate, a protecting group is removed and, if desired, a compound obtained is converted into a pharmaceutically acceptable salt thereof or, if desired, a salt of is converted into a free base.

**2.** A process of Claim 1 in which $R^1$ and $R^2$ are, independently, hydrogen, fluoro, hydroxy, alkoxy of 1 to 6 carbon atoms or alkanoyloxy of 2 to 6 carbon atoms, or together form a methylenedioxy, ethylenedioxy or propylenedioxy ring; $R^4$ and $R^5$ are, independently, hydrogen or alkyl of 1 to 6 carbon atoms; and the bond between oxygen and the anilino moiety is in meta position, or the $-(CH_2)_n$-O- link is to the 7-position of the coumarin or carbostyril nucleus.

**3.** A process as claimed in Claim 1 carried to prepare a compound of the formula

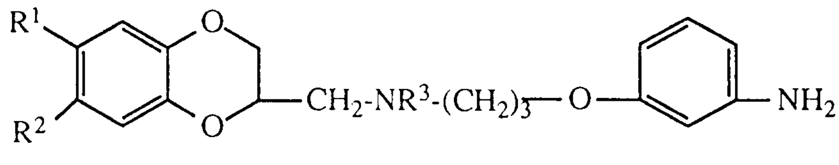

in which

$R^1$ and $R^2$ are, independently, hydrogen, fluoro, hydroxy, alkoxy of 1 to 4 carbon atoms, alkanoyloxy of 2 to 4 carbon atoms or together form an alkylenedioxy ring of 1 to 3 carbon atoms;

$R^3$ is hydrogen or alkyl of 1 to 4 carbon atoms;
or a pharmaceutically acceptable salt thereof, where the benzodioxan methanamine moiety is in the S-configuration.

**4.** A process as claimed in Claim 1 carried out to prepare a compound of the formula

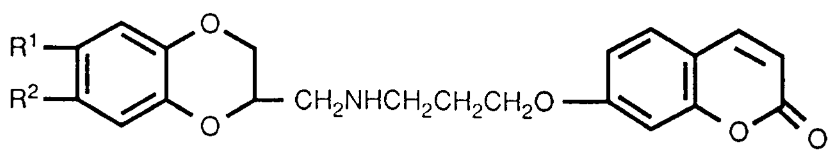

in which $R^1$ and $R^2$ are, independently, hydrogen, fluoro, hydroxy, alkoxy of 1 to 6 carbon atoms, alkanoyloxy of 2 to 6 carbon atoms, or, taken together, $R^1$ and $R^2$ are methylenedioxy, ethylenedioxy or propylenedioxy, or a pharmaceutically acceptable salt thereof, wherein the benzodioxan methamine moiety is in the S-configuration.

**5.** A process as claimed in claim 1 carried out to prepare a compound selected from
N-[3-(3-aminophenoxy)propyl]-2,3-dihydro-7-hydroxy-8-methyl-1,4-benzodioxin-2-methanamine;
N-[3-(3-aminophenoxy)propyl]-2,3-dihydro-6,8-dimethoxy-1,4-benzodioxin-2-methanamine; N-[3-(3-aminophenoxy)propyl]-2,3-dihydro-7-fluoro-1,4-benzodioxin-2-methanamine;
N-[3-(3-aminophenoxy)propyl]-2,3-dihydro-5,6-dimethoxy-1,4-benzodioxin-2-methanamine;
N-[3-(3-aminophenoxy)propyl]-2,3-dihydro-6,7-ethylenedioxy-1,4-benzodioxin-2-methanamine; N-[3-(3-aminophenoxy)propyl]-2,3-dihydro-6,7-dimethoxy-1,4-benzodioxin-2-methanamine; N-[3-(3-aminophenoxy)propyl]-2,3-dihydro-7-chloro-1,4-benzodioxin-2-methanamine;
N-[3-(3-aminophenoxy)propyl]-2,3-dihydro-8-hydroxy-1,4-benzodioxin-2-methanamine;
N-[3-(3-aminophenoxy)propyl]-2,3-dihydro-8-methoxy-1,4-benzodioxin-2-methanamine;
N-[3-(3-aminophenoxy)propyl]-2,3-dihydro-6,7-methylenedioxy-1,4-benzodioxin-2-methanamine; N-[3-(3-aminophenoxy)propyl]-2,3-dihydro-7-methoxy-1,4-benzodioxin-2-methanamine;
N-[3-(3-aminophenoxy)propyl]-2,3-dihydro-6-methyl-7-methoxy-1,4-benzodioxin-2-methanamine;
N-[3-(3-aminophenoxy)propyl]-2,3-dihydro-5-hydroxy-1,4-benzodioxin-2-methanamine;
N-[3-(3-aminophenoxy)propyl]-2,3-dihydro-6-hydroxy-1,4-benzodioxin-2-methanamine;
N-[3-(3-aminophenoxy)propyl]-2,3-dihydro-7-hydroxy-1,4-benzodioxin-2-methanamine;
N-[3-(3-aminophenoxy)propyl]-2,3-dihydro-6-methyl-7-hydroxy-1,4-benzodioxin-2-methanamine;
N-[3-(3-aminophenoxy)propyl]-2,3-dihydro-5-methoxy-1,4-benzodioxin-2-methanamine;
and the pharmaceutically acceptable salts thereof.

**6.** A process as claimed in claim 1 carried out so as to prepare a compound selected from
(R)-N-[3-(3-aminophenoxy)propyl]-2,3-dihydro-7-hydroxy-1,4-benzodioxin-2-methanamine;
(R)-N-[3-(3-aminophenoxy)propyl]-2,3-dihydro-6,7-methylenedioxy-1,4-benzodioxin-2-methanamine;
(R)-N-[3-(3-aminophenoxy)propyl]-2,3-dihydro-7-fluoro-1,4-benzodioxin-2-methanamine;
(R)-N-[3-(3-aminophenoxy)propyl]-2,3-dihydro-7-methoxy-1,4-benzodioxin-2-methanamine;
and the pharmaceutically acceptable salts thereof.

**7.** A process as claimed in claim 1, carried out to prepare a compound selected from
(S)-N-[3-(3-aminophenoxy)propyl]-2,3-dihydro-7-hydroxy-1,4-benzodioxin-2-methanamine;
(S)-N-[3-(3-aminophenoxy)propyl]-2,3-dihydro-6,7-methylenedioxy-1,4-benzodioxin-2-methanamine;
(S)-N-[3-(3-aminophenoxy)propyl]-2,3-dihydro-7-fluoro-1,4-benzodioxin-2-methanamine;
(S)-N-[3-(3-aminophenoxy)propyl]-2,3-dihydro-7-methoxy-1,4-benzodioxin-2-methanamine;
and the pharmaceutically acceptable salts thereof.

**8.** A process as claimed in claim 1, carried out to prepare a compound selected from
7-[3-[[(2,3-dihydro-1,4-benzodioxin-2-yl)methyl]amino] propoxy]-2H-1-benzopyran-2-one;
7-[3-[[(2,3-dihydro-6,8-methoxy-1,4-benzodioxin-2-yl) methyl]amino]propoxy]-2H-1-benzopyran-2-one;
7-[3-[[(2,3-dihydro-5-methoxy-1,4-benzodioxin-2-yl) methyl]amino]propoxy]-2H-1-benzopyran-2-one;
7-[3-[[(2,3-dihydro-7-chloro-1,4-benzodioxin-2-yl) methyl]amino]propoxy]-2H-1-benzopyran-2-one;

7-[3-[[(2,3-dihydro-7-phenylmethoxy-1,4-benzodioxin-2-yl)methyl]amino]propoxy]-2H-1-benzopyran-2-one;
7-[3-[[(2,3-dihydro-6-phenylmethoxy-1,4-benzodioxin-2-yl)methyl]amino]propoxy]-2H-1-benzopyran-2-one;
7-[3-[[(2,3-dihydro-6-hydroxy-1,4-benzodioxin-2-yl)methyl]amino]propoxy]-2H-1-benzopyran-2-one;
7-[3-[[(2,3-dihydro-7-hydroxy-1,4-benzodioxin-2-yl)methyl]amino]propoxy]-2H-1-benzopyran-2-one;
7-[3-[[(2,3-dihydro-7-methoxy-1,4-benzodioxin-2-yl)methyl]amino]propoxy]-2H-1-benzopyran-2-one;
7-[3-[[(2,3-dihydro-6-acetoxy-1,4-benzodioxin-2-yl)methyl]amino]propoxy]-2H-1-benzopyran-2-one;
7-[3-[[(2,3-dihydro-7-hydroxy-1,4-benzodioxin-2-yl) methyl]ethylamino]propoxy]-2H-1-benzopyran-2-one;
7-[3-[[(2,3-dihydro-1,4-benzodioxin-2-yl) methyl]ethylamino]propoxy]-2H-1-benzopyran-2-one;
7-[3-[[(2,3-dihydro-7-methoxy-1,4-benzodioxin-2-yl) methyl]methylamino]propoxy]-2H-1-benzopyran-2-one;
7-[3-[[(2,3-dihydro-6-hydroxy-1,4-benzodioxin-2-yl) methyl]methylamino]propoxy]-2H-1-benzopyran-2-one;
and the pharmaceutically acceptable salts thereof.

**9.** A process as claimed in claim 1 carried out to prepare a compound selected from
(S)-7-[3-[[(2,3-dihydro-7-hydroxy-1,4-benzodioxin-2-yl)methyl]ethylamino]propoxy]-2H-1-benzopyran-2-one;
(S)-7-[3-[[(2,3-dihydro-7-hydroxy-1,4-benzodioxin-2-yl)methyl]amino]propoxy]-2H-1-benzopyran-2-one;
(S)-7-[3-[[(2,3-dihydro-7-methoxy-1,4-benzodioxin-2-yl)methyl]amino]propoxy]-2H-1-benzopyran-2-one;
(S)-7-[3-[[(2,3-dihydro-6,7-methylenedioxy-1,4-benzodioxin-2-yl)methyl]ethylamino]propoxy]-2H-1- benzopyran-2-one;
(S)-7-[3-[[(2,3-dihydro-6,7-methylenedioxy-1,4-benzodioxin-2-yl)methyl]amino]propoxy]-2H-1- benzopyran-2-one;
(S)-7-[3-[[(2,3-dihydro-6-hydroxy-1,4-benzodioxin-2-yl)methyl]amino]propoxy]-2H-1-benzopyran-2-one;
and their pharmaceutically acceptable salts.

**10.** A process as claimed in claim 1 carried out to prepare a compound selected from
5-[3[[(2,3-dihydro-1,4-benzodioxin-2-yl)methyl]amino] propoxy]-2(1H)-quinoline;
(R)-7-[3-[[(2,3-dihydro-6-hydroxy-1,4-benzodioxin-2-yl) methyl)amino]propoxy]-2H-1-benzopyran-2-one
and their pharmaceutically acceptable salts.

**11.** A process of making a pharmaceutical preparation characterized by bringing a compound having the formula

$$Y^1\text{-}CH_2\text{-}NR^3\text{-}CH_2\text{-}Y^2$$

where $Y^1$, $R^3$ and $Y^2$ are as defined in claim 1 or a pharmaceutically acceptable salt thereof into combination or association with a pharmaceutical carrier.

**12.** Use of a compound having the formula

$$Y^1\text{-}CH_2\text{-}NR^3\text{-}CH_2\text{-}Y^2$$

where $Y^1$, $R^3$ and $Y^2$ are as defined in claim 1 or a pharmaceutically acceptable salt thereof to prepare a medicament for use as a dopamine autoreceptor agonist.

**Patentansprüche**

**Patentansprüche für folgende Vertragsstaaten : AT, BE, CH, DE, DK, FR, GB, IT, LI, LU, NL, PT, SE**

1. Verbindung der Formel:

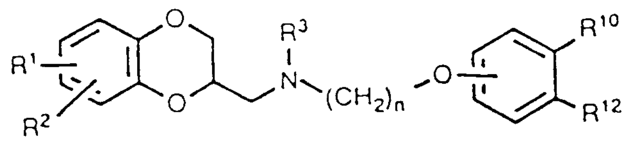

,

worin $R^1$ und $R^2$ unabhängig Wasserstoff, Alkyl mit 1 bis 6 Kohlenstoffatomen, Alkoxy mit 1 bis 6 Kohlenstoffatomen, Aralkoxy mit 7 bis 12 Kohlenstoffatomen, Alkanoyloxy mit 2 bis 6 Kohlenstoffatomen, Hydroxy, Halogen, Amino, Mono- oder Dialkylamino, wobei jede Allyl-Gruppe 1 bis 6 Kohlenstoffatome enthält, Alkanamido mit 2 bis 6 Kohlenstoffatomen oder Sulfonamido bedeuten, oder $R^1$ und $R^2$ zusammen Methylendioxy, Ethylendioxy oder Propylendioxy bilden; $R^3$ Wasserstoff oder Alkyl mit 1 bis 6 Kohlenstoffatomen darstellt; n eine der ganzen Zahlen 2, 3 oder 4 ist; $R^{12}$ die Bedeutung $NR^4R^5$ hat, wobei $R^4$ und $R^5$ unabhängig Wasserstoff, Alkyl mit 1 bis 6 Kohlenstoffatomen, Cycloalkyl mit 4 bis 7 Kohlenstoffatomen, Alkanoyl mit 2 bis 6 Kohlenstoffatomen, Aroyl mit 7 bis 12 Kohlenstoffatomen, Alkylsulfonyl mit 1 bis 6 Kohlenstoffatomen oder Arylsulfonyl mit 6 bis 10 Kohlenstoffatomen darstellen, oder $R^4$ und $R^5$ zusammen einen 3- bis 7-gliedrigen Polymethylen-Ring bilden; $R^{10}$ Wasserstoff ist, oder,
zusammen mit $R^{12}$, $R^{10}$ und $R^{12}$ den 6-gliedrigen Ring -

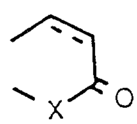

vervollständigen, worin X die Bedeutung O oder $NR^8$ hat, wobei $R^8$ Wasserstoff oder Alkyl mit 1 bis 6 Kohlenstoffatomen darstellt; oder ein pharmazeutisch annehmbares Salz hievon.

2. Verbindung nach Anspruch 1, worin $R^1$ und $R^2$ unabhängig Wasserstoff, Fluor, Hydroxy, Alkoxy mit 1 bis 6 Kohlenstoffatomen oder Alkanoyloxy mit 2 bis 6 Kohlenstoffatomen bedeuten, oder zusammen einen Methylendioxy-, Ethylendioxy- oder Propylendioxy-Ring bilden; $R^4$ und $R^5$ unabhängig Wasserstoff oder Alkyl mit 1 bis 6 Kohlenstoffatomen darstellen; und die Bindung zwischen Sauerstoff und der Anilino-Gruppe in meta-Stellung vorliegt, oder die $-(CH_2)_n$-O-Bindung in Stellung 7 des Coumarin- oder Carbostyril-Kerns vorliegt; oder ein pharmazeutisch annehmbares Salz hievon.

3. Verbindung nach Anspruch 1 der Formel:

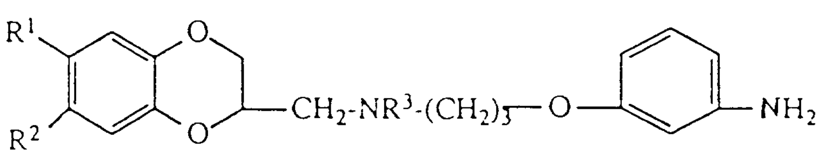

,

worin $R^1$ und $R^2$ unabhängig Wasserstoff, Fluor, Hydroxy, Alkoxy mit 1 bis 4 Kohlenstoffatomen, Alkanoyloxy mit 2 bis 4 Kohlenstoffatomen bedeuten, oder zusammen einen Alkylendioxy-Ring mit 1 bis 3 Kohlenstoffatomen bilden; und $R^3$ Wasserstoff oder Alkyl mit 1 bis 4 Kohlenstoffatomen darstellt; oder ein pharmazeutisch annehmbares Salz hievon, wobei die Benzodioxanmethanamin-Gruppe in S-Konfiguration vorliegt.

4. Verbindung nach Anspruch 1 der Formel:

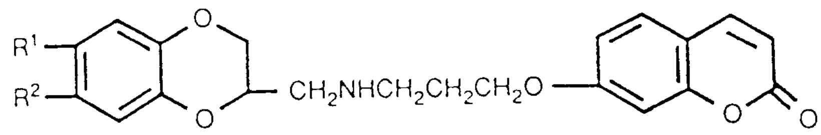

,

worin $R^1$ und $R^2$ unabhängig Wasserstoff, Fluor, Hydroxy, Alkoxy mit 1 bis 6 Kohlenstoffatomen, Alkanoyloxy mit 2 bis 6 Kohlenstoffatomen bedeuten, oder, zusammen, $R^1$ und $R^2$ Methylendioxy, Ethylendioxy oder Propylendioxy darstellen; oder ein pharmazeutisch annehmbares Salz hievon, wobei die Benzodioxanmethamin-Gruppe in S-Konfiguration vorliegt.

5. Verbindung nach Anspruch 1, welche ausgewählt ist aus:
N-[3-(3-Aminophenoxy)-propyl]-2,3-dihydro-7-hydroxy-8-methyl-1,4-benzodioxin-2-methanamin;
N-[3-(3-Aminophenoxy)-propyl]-2,3-dihydro-6,8-dimethoxy-1,4-benzodioxin-2-methanamin;
N-[3-(3-Aminophenoxy)-propyl]-2,3-dihydro-7-fluor-1,4-benzodioxin-2-methanamin;
N-[3-(3-Aminophenoxy)-propyl]-2,3-dihydro-5,6-dimethoxy-1,4-benzodioxin-2-methanamin;
N-[3-(3-Aminophenoxy)-propyl]-2,3-dihydro-6,7-ethylendioxy-1,4-benzodioxin-2-methanamin;
N-[3-(3-Aminophenoxy)-propyl]-2,3-dihydro-6,7-dimethoxy-1,4-benzodioxin-2-methanamin;
N-[3-(3-Aminophenoxy)-propyl]-2,3-dihydro-7-chlor-1,4-benzodioxin-2-methanamin;
N-[3-(3-Aminophenoxy)-propyl]-2,3-dihydro-8-hydroxy-1,4-benzodioxin-2-methanamin;
N-[3-(3-Aminophenoxy)-propyl]-2,3-dihydro-8-methoxy-1,4-benzodioxin-2-methanamin;
N-[3-(3-Aminophenoxy)-propyl]-2,3-dihydro-6,7-methylendioxy-1,4-benzodioxin-2-methanamin;
N-[3-(3-Aminophenoxy)-propyl]-2,3-dihydro-7-methoxy-1,4-benzodioxin-2-methanamin;
N-[3-(3-Aminophenoxy)-propyl]-2,3-dihydro-6-methyl-7-methoxy-1,4-benzodioxin-2-methanamin;
N-[3-(3-Aminophenoxy)-propyl]-2,3-dihydro-5-hydroxy-1,4-benzodioxin-2-methanamin;
N-[3-(3-Aminophenoxy)-propyl]-2,3-dihydro-6-hydroxy-1,4-benzodioxin-2-methanamin;
N-[3-(3-Aminophenoxy)-propyl]-2,3-dihydro-7-hydroxy-1,4-benzodioxin-2-methanamin;
N-[3-(3-Aminophenoxy)-propyl]-2,3-dihydro-6-methyl-7-hydroxy-1,4-benzodioxin-2-methanamin;
N-[3-(3-Aminophenoxy)-propyl]-2,3-dihydro-5-methoxy-1,4-benzodioxin-2-methanamin;
und den pharmazeutisch annehmbaren Salzen hievon.

6. Verbindung nach Anspruch 1, welche ausgewählt ist aus:
(R)-N-[3-(3-Aminophenoxy)-propyl]-2,3-dihydro-7-hydroxy-1,4-benzodioxin-2-methanamin;
(R)-N-[3-(3-Aminophenoxy)-propyl]-2,3-dihydro-6,7-methylendioxy-1,4-benzodioxin-2-methanamin;
(R)-N-[3-(3-Aminophenoxy)-propyl]-2,3-dihydro-7-fluor-1,4-benzodioxin-2-methanamin;
(R)-N-[3-(3-Aminophenoxy)-propyl]-2,3-dihydro-7-methoxy-1,4-benzodioxin-2-methanamin;
und den pharmazeutisch annehmbaren Salzen hievon.

7. Verbindung nach Anspruch 1, welche ausgewählt ist aus:
(S)-N-[3-(3-Aminophenoxy)-propyl]-2,3-dihydro-7-hydroxy-1,4-benzodioxin-2-methanamin;
(S)-N-[3-(3-Aminophenoxy)-propyl]-2,3-dihydro-6,7-methylendioxy-1,4-benzodioxin-2-methanamin;
(S)-N-[3-(3-Aminophenoxy)-propyl]-2,3-dihydro-7-fluor-1,4-benzodioxin-2-methanamin;
(S)-N-[3-(3-Aminophenoxy)-propyl]-2,3-dihydro-7-methoxy-1,4-benzodioxin-2-methanamin;
und den pharmazeutisch annehmbaren Salzen hievon.

8. Verbindung nach Anspruch 1, welche ausgewählt ist aus:
7-[3-[[(2,3-Dihydro-1,4-benzodioxin-2-yl)-methyl]-amino]-propoxy]-2H-1-benzopyran-2-on;
7-[3-[[(2,3-Dihydro-6,8-methoxy-1,4-benzodioxin-2-yl)-methyl]-amino]-propoxy]-2H-1-benzopyran-2-on;
7-[3-[[(2,3-Dihydro-5-methoxy-1,4-benzodioxin-2-yl)-methyl]-amino]-propoxy]-2H-1-benzopyran-2-on;
7-[3-[[(2,3-Dihydro-7-chlor-1,4-benzodioxin-2-yl)-methyl]-amino]-propoxy]-2H-1-benzopyran-2-on;
7-[3-[[(2,3-Dihydro-7-phenylmethoxy-1,4-benzodioxin-2-yl)-methyl]-amino]-propoxy]-2H-1-benzopyran-2-on;
7-[3-[[(2,3-Dihydro-6-phenylmethoxy-1,4-benzodioxin-2-yl)-methyl]-amino]-propoxy]-2H-1-benzopyran-2-on;
7-[3-[[(2,3-Dihydro-6-hydroxy-1,4-benzodioxin-2-yl)-methyl]-amino]-propoxy]-2H-1-benzopyran-2-on;

7-[3-[[(2,3-Dihydro-7-hydroxy-1,4-benzodioxin-2-yl)-methyl]-amino]-propoxy]-2H-1-benzopyran-2-on;
7-[3-[[(2,3-Dihydro-7-methoxy-1,4-benzodioxin-2-yl)-methyl]-amino]-propoxy]-2H-1-benzopyran-2-on;
7-[3-[[(2,3-Dihydro-6-acetoxy-1,4-benzodioxin-2-yl)-methyl]-amino]-propoxy]-2H-1-benzopyran-2-on;
7-[3-[[(2,3-Dihydro-7-hydroxy-1,4-benzodioxin-2-yl)-methyl]-ethylamino]-propoxy]-2H-1-benzopyran-2-on;
7-[3-[[(2,3-Dihydro-1,4-benzodioxin-2-yl)-methyl]-ethylamino]-propoxy]-2H-1-benzopyran-2-on;
7-[3-[[(2,3-Dihydro-7-methoxy-1,4-benzodioxin-2-yl)-methyl]-methylamino]-propoxy]-2H-1-benzopyran-2-on;
7-[3-[[(2,3-Dihydro-6-hydroxy-1,4-benzodioxin-2-yl)-methyl]-methylamino]-propoxy]-2H-1-benzopyran-2-on;
und den pharmazeutisch annehmbaren Salzen hievon.

**9.** Verbindung nach Anspruch 1, welche ausgewählt ist aus:
(S)-7-[3-[[(2,3-Dihydro-7-hydroxy-1,4-benzodioxin-2-yl)-methyl]-ethylamino]-propoxy]-2H-1-benzopyran-2-on;
(S)-7-[3-[[(2,3-Dihydro-7-hydroxy-1,4-benzodioxin-2-yl)-methyl]-amino]-propoxy]-2H-1-benzopyran-2-on;
(S)-7-[3-[[(2,3-Dihydro-7-methoxy-1,4-benzodioxin-2-yl)-methyl]-amino]-propoxy]-2H-1-benzopyran-2-on;
(S)-7-[3-[[(2,3-Dihydro-6,7-methylendioxy-1,4-benzodioxin-2-yl)-methyl]-ethylamino]-propoxy]-2H-1-benzopyran-2-on;
(S)-7-[3-[[(2,3-Dihydro-6,7-methylendioxy-1,4-benzodioxin-2-yl)-methyl]-amino]-propoxy]-2H-1-benzopyran-2-on;
(S)-7-[3-[[(2,3-Dihydro-6-hydroxy-1,4-benzodioxin-2-yl)-methyl]-amino]-propoxy]-2H-1-benzopyran-2-on;
und ihren pharmazeutisch annehmbaren Salzen.

**10.** Verbindung nach Anspruch 1, welche ausgewählt ist aus:
5-[3-[[2,3-Dihydro-1,4-benzodioxin-2-yl)-methyl]-amino]-propoxy]-2(1H)-chinolin;
(R)-7-[3-[[(2,3-Dihydro-6-hydroxy-1,4-benzodioxin-2-yl)-methyl]-amino]-propoxy]-2H-1-benzopyran-2-on;
und ihren pharmazeutisch annehmbaren Salzen.

**11.** Pharmazeutische Zusammensetzung, welche eine Verbindung nach einem der Ansprüche 1 bis 10 in Kombination oder Vereinigung mit einem pharmazeutischen Träger umfaßt.

**Patentansprüche für folgende Vertragsstaaten : ES, GR**

**1.** Verfahren zur Herstellung einer Verbindung der Formel:

$Y^1$-$CH_2$-$NR^3$-$CH_2$-$Y^2$

oder eines pharmazeutisch annehmbaren Salzes hievon, wobei $R^3$ Wasserstoff oder Alkyl mit 1 bis 6 Kohlenstoffatomen bedeutet; eines von $Y^1$ und $Y^2$ eine Gruppe darstellt der Formel:

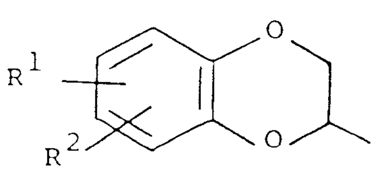

,

worin $R^1$ und $R^2$ unabhängig Wasserstoff, Alkyl mit 1 bis 6 Kohlenstoffatomen, Alkoxy mit 1 bis 6 Kohlenstoffatomen, Aralkoxy mit 7 bis 12 Kohlenstoffatomen, Alkanoyloxy mit 2 bis 6 Kohlenstoffatomen, Hydroxy, Halogen, Amino, Mono- oder Dialkylamino, wobei jede Alkyl-Gruppe 1 bis 6 Kohlenstoffatome enthält, Alkanamido mit 2 bis 6 Kohlenstoffatomen, Sulfonamido bedeuten, oder $R^1$ und $R^2$ zusammen Methylendioxy, Ethylendioxy oder Propylendioxy bilden; und das andere von $Y^1$ und $Y^2$ eine Gruppe darstellt der Formel:

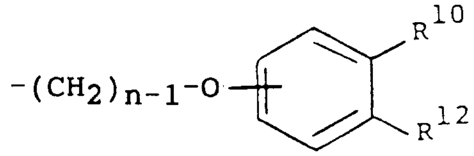

,

worin n eine der ganzen Zahlen 2, 3 oder 4 ist; $R^{12}$ die Bedeutung $NR^4R^5$ hat, wobei $R^4$ und $R^5$ unabhängig Wasserstoff, Alkyl mit 1 bis 6 Kohlenstoffatomen, Cycloalkyl mit 4 bis 7 Kohlenstoffatomen, Alkanoyl mit 2 bis 6 Kohlenstoffatomen, Aroyl mit 7 bis 12 Kohlenstoffatomen, Alkylsulfonyl mit 1 bis 6 Kohlenstoffatomen oder Arylsulfonyl mit 6 bis 10 Kohlenstoffatomen darstellen, oder $R^4$ und $R^5$ zusammen einen 3- bis 7-gliedrigen Polymethylen-Ring bilden; $R^{10}$ Wasserstoff ist, oder, zusammen mit $R^{12}$, $R^{10}$ und $R^{12}$ den 6-gliedrigen Ring

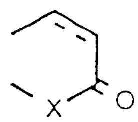

vervollständigen, worin die strichlierte Linie eine gegebenenfalls vorhandene Unsättigung anzeigt, und X die Bedeutung O oder $NR^8$ hat, wobei $R^8$ Wasserstoff oder Alkyl mit 1 bis 6 Kohlenstoffatomen darstellt; bei welchem Verfahren:

(a) ein Amin der Formel

$Y^1$-$CH_2$-$NHR^3$

N-alkyliert wird, zur Einführung einer substituierten Alkyl-Gruppe der Formel -$CH_2$-$Y^2$, worin $Y^1$, $Y^2$ und $R^3$ wie oben definiert sind, außer daß eine Amino- oder Monoalkylamino-Gruppe, dargestellt durch $R^1$, $R^2$ oder -$NR^4R^5$, oder eine Hydroxy-Gruppe, dargestellt durch $R^1$ oder $R^2$, vorübergehend durch eine entfernbare Schutzgruppe geschützt werden kann; oder

(b) ein Amin der Formel

$Y^1$-$CH_2$-$NHR^3$

mit einem Aldehyd der Formel $Y^2$-CHO, worin $Y^1$, $Y^2$ und $R^3$ wie oben definiert sind, reduktiv N-alkyliert wird; oder

(c) ein Amin der Formel

$Y^1$-CO-$NR^3$-$CH_2$-$Y^2$ ,

worin $Y^1$, $Y^2$ und $R^3$ wie oben definiert sind, reduziert wird, wobei die Carbonyl-Gruppe hievon in Methylen übergeführt wird; oder

(d) eine Nitro-Verbindung der Formel:

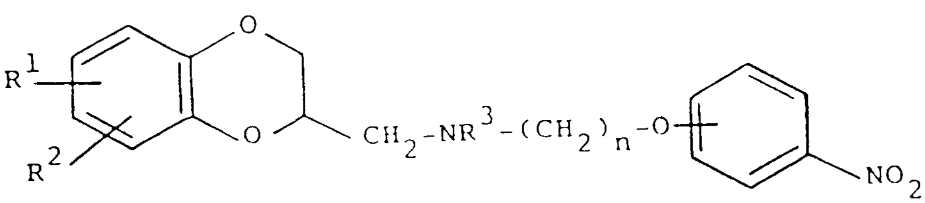

,

worin $R^1$, $R^2$, $R^3$ und n wie oben definiert sind, reduziert wird, wobei die Nitro-Gruppe in Amino übergeführt wird; oder

(e) ein Amin der Formel:

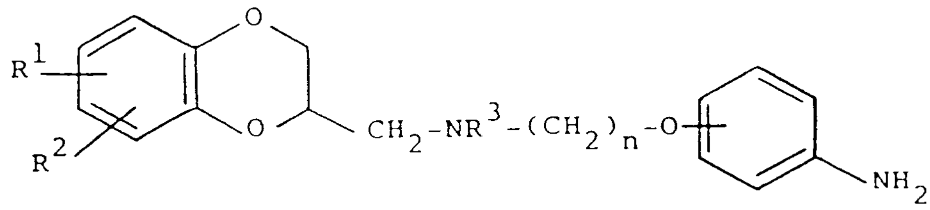

einer Alkylierung, Acylierung oder Alkyl- oder Arylsulfonierung unterworfen wird, wobei $-NH_2$ in eine andere Bedeutung von $-NR^4R^5$ übergeführt wird; und, wenn angebracht, eine Schutzgruppe entfernt wird, und, wenn gewünscht, eine erhaltene Verbindung in ein pharmazeutisch annehmbares Salz hievon übergeführt wird, oder, wenn gewünscht, ein Salz in eine freie Base übergeführt wird.

**2.** Verfahren nach Anspruch 1, wobei $R^1$ und $R^2$ unabhängig Wasserstoff, Fluor, Hydroxy, Alkoxy mit 1 bis 6 Kohlenstoffatomen oder Alkanoyloxy mit 2 bis 6 Kohlenstoffatomen bedeuten, oder zusammen einen Methylendioxy-, Ethylendioxy- oder Propylendioxy-Ring bilden; $R^4$ und $R^5$ unabhängig Wasserstoff oder Alkyl mit 1 bis 6 Kohlenstoffatomen darstellen; und die Bindung zwischen Sauerstoff und der Anilino-Gruppe in meta-Stellung vorliegt, oder die $-(CH_2)_n-O-$Bindung in Stellung 7 des Coumarin- oder Carbostyril-Kerns vorliegt.

**3.** Verfahren nach Anspruch 1, welches durchgeführt wird zur Herstellung einer Verbindung der Formel:

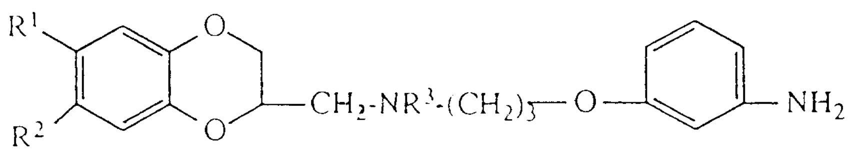

,

worin $R^1$ und $R^2$ unabhängig Wasserstoff, Fluor, Hydroxy, Alkoxy mit 1 bis 4 Kohlenstoffatomen, Alkanoyloxy mit 2 bis 4 Kohlenstoffatomen bedeuten, oder zusammen einen Alkylendioxy-Ring mit 1 bis 3 Kohlenstoffatomen bilden; und $R^3$ Wasserstoff oder Alkyl mit 1 bis 4 Kohlenstoffatomen darstellt; oder eines pharmazeutisch annehmbaren Salzes hievon, wobei die Benzodioxanmethanamin-Gruppe in S-Konfiguration vorliegt.

**4.** Verfahren nach Anspruch 1, welches durchgeführt wird zur Herstellung einer Verbindung der Formel:

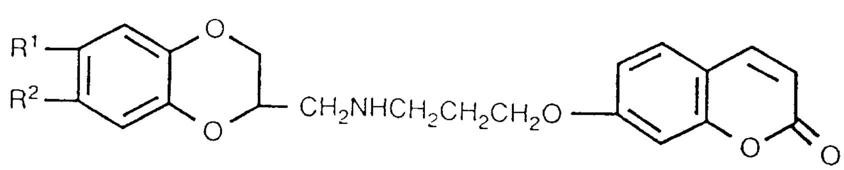

,

worin $R^1$ und $R^2$ unabhängig Wasserstoff, Fluor, Hydroxy, Alkoxy mit 1 bis 6 Kohlenstoffatomen, Alkanoyloxy mit 2 bis 6 Kohlenstoffatomen bedeuten, oder, zusammen, $R^1$ und $R^2$ Methylendioxy, Ethylendioxy oder Propylendioxy darstellen; oder eines pharmazeutisch annehmbaren Salzes hievon, wobei die Benzodioxanmethamin-Gruppe in S-Konfiguration vorliegt.

**5.** Verfahren nach Anspruch 1, welches durchgeführt wird zur Herstellung einer Verbindung, ausgewählt aus:
N-[3-(3-Aminophenoxy)-propyl]-2,3-dihydro-7-hydroxy-8-methyl-1,4-benzodioxin-2-methanamin;
N-[3-(3-Aminophenoxy)-propyl]-2,3-dihydro-6,8-dimethoxy-1,4-benzodioxin-2-methanamin;
N-[3-(3-Aminophenoxy)-propyl]-2,3-dihydro-7-fluor-1,4-benzodioxin-2-methanamin;
N-[3-(3-Aminophenoxy)-propyl]-2,3-dihydro-5,6-dimethoxy-1,4-benzodioxin-2-methanamin;

N-[3-(3-Aminophenoxy)-propyl]-2,3-dihydro-6,7-ethylendioxy-1,4-benzodioxin-2-methanamin;
N-[3-(3-Aminophenoxy)-propyl]-2,3-dihydro-6,7-dimethoxy-1,4-benzodioxin-2-methanamin;
N-[3-(3-Aminophenoxy)-propyl]-2,3-dihydro-7-chlor-1,4-benzodioxin-2-methanamin;
N-[3-(3-Aminophenoxy)-propyl]-2,3-dihydro-8-hydroxy-1,4-benzodioxin-2-methanamin;
N-[3-(3-Aminophenoxy)-propyl]-2,3-dihydro-8-methoxy-1,4-benzodioxin-2-methanamin;
N-[3-(3-Aminophenoxy)-propyl]-2,3-dihydro-6,7-methylendioxy-1,4-benzodioxin-2-methanamin;
N-[3-(3-Aminophenoxy)-propyl]-2,3-dihydro-7-methoxy-1,4-benzodioxin-2-methanamin;
N-[3-(3-Aminophenoxy)-propyl]-2,3-dihydro-6-methyl-7-methoxy-1,4-benzodioxin-2-methanamin;
N-[3-(3-Aminophenoxy)-propyl]-2,3-dihydro-5-hydroxy-1,4-benzodioxin-2-methanamin;
N-[3-(3-Aminophenoxy)-propyl]-2,3-dihydro-6-hydroxy-1,4-benzodioxin-2-methanamin;
N-[3-(3-Aminophenoxy)-propyl]-2,3-dihydro-7-hydroxy-1,4-benzodioxin-2-methanamin;
N-[3-(3-Aminophenoxy)-propyl]-2,3-dihydro-6-methyl-7-hydroxy-1,4-benzodioxin-2-methanamin;
N-[3-(3-Aminophenoxy)-propyl]-2,3-dihydro-5-methoxy-1,4-benzodioxin-2-methanamin;
und den pharmazeutisch annehmbaren Salzen hievon.

**6.** Verfahren nach Anspruch 1, welches durchgeführt wird zur Herstellung einer Verbindung, ausgewählt aus:
(R)-N-[3-(3-Aminophenoxy)-propyl]-2,3-dihydro-7-hydroxy-1,4-benzodioxin-2-methanamin;
(R)-N-[3-(3-Aminophenoxy)-propyl]-2,3-dihydro-6,7-methylendioxy-1,4-benzodioxin-2-methanamin;
(R)-N-[3-(3-Aminophenoxy)-propyl]-2,3-dihydro-7-fluor-1,4-benzodioxin-2-methanamin;
(R)-N-[3-(3-Aminophenoxy)-propyl]-2,3-dihydro-7-methoxy-1,4-benzodioxin-2-methanamin;
und den pharmazeutisch annehmbaren Salzen hievon.

**7.** Verfahren nach Anspruch 1, welches durchgeführt wird zur Herstellung einer Verbindung, ausgewählt aus:
(S)-N-[3-(3-Aminophenoxy)-propyl]-2,3-dihydro-7-hydroxy-1,4-benzodioxin-2-methanamin;
(S)-N-[3-(3-Aminophenoxy)-propyl]-2,3-dihydro-6,7-methylendioxy-1,4-benzodioxin-2-methanamin;
(S)-N-[3-(3-Aminophenoxy)-propyl]-2,3-dihydro-7-fluor-1,4-benzodioxin-2-methanamin;
(S)-N-[3-(3-Aminophenoxy)-propyl]-2,3-dihydro-7-methoxy-1,4-benzodioxin-2-methanamin;
und den pharmazeutisch annehmbaren Salzen hievon.

**8.** Verfahren nach Anspruch 1, welches durchgeführt wird zur Herstellung einer Verbindung, ausgewählt aus:
7-[3-[[(2,3-Dihydro-1,4-benzodioxin-2-yl)-methyl]-amino]-propoxy]-2H-1-benzopyran-2-on;
7-[3-[[(2,3-Dihydro-6,8-methoxy-1,4-benzodioxin-2-yl)-methyl]-amino]-propoxy]-2H-1-benzopyran-2-on;
7-[3-[[(2,3-Dihydro-5-methoxy-1,4-benzodioxin-2-yl)-methyl]-amino]-propoxy]-2H-1-benzopyran-2-on;
7-[3-[[(2,3-Dihydro-7-chlor-1,4-benzodioxin-2-yl)-methyl]-amino]-propoxy]-2H-1-benzopyran-2-on;
7-[3-[[(2,3-Dihydro-7-phenylmethoxy-1,4-benzodioxin-2-yl)-methyl]-amino]-propoxy]-2H-1-benzopyran-2-on;
7-[3-[[(2,3-Dihydro-6-phenylmethoxy-1,4-benzodioxin-2-yl)-methyl]-amino]-propoxy]-2H-1-benzopyran-2-on;
7-[3-[[(2,3-Dihydro-6-hydroxy-1,4-benzodioxin-2-yl)-methyl]-amino]-propoxy]-2H-1-benzopyran-2-on;
7-[3-[[(2,3-Dihydro-7-hydroxy-1,4-benzodioxin-2-yl)-methyl]-amino]-propoxy]-2H-1-benzopyran-2-on;
7-[3-[[(2,3-Dihydro-7-methoxy-1,4-benzodioxin-2-yl)-methyl]-amino]-propoxy]-2H-1-benzopyran-2-on;
7-[3-[[(2,3-Dihydro-6-acetoxy-1,4-benzodioxin-2-yl)-methyl]-amino]-propoxy]-2H-1-benzopyran-2-on;
7-[3-[[(2,3-Dihydro-7-hydroxy-1,4-benzodioxin-2-yl)-methyl]-ethylamino]-propoxy]-2H-1-benzopyran-2-on;
7-[3-[[(2,3-Dihydro-1,4-benzodioxin-2-yl)-methyl]-ethylamino]-propoxy]-2H-1-benzopyran-2-on;
7-[3-[[(2,3-Dihydro-7-methoxy-1,4-benzodioxin-2-yl)-methyl]-methylamino]-propoxy]-2H-1-benzopyran-2-on;
7-[3-[[(2,3-Dihydro-6-hydroxy-1,4-benzodioxin-2-yl)-methyl]-methylamino]-propoxy]-2H-1-benzopyran-2-on;
und den pharmazeutisch annehmbaren Salzen hievon.

**9.** Verfahren nach Anspruch 1, welches durchgeführt wird zur Herstellung einer Verbindung, ausgewählt aus:
(S)-7-[3-[[(2,3-Dihydro-7-hydroxy-1,4-benzodioxin-2-yl)-methyl]-ethylamino]-propoxy]-2H-1-benzopyran-2-on;

(S)-7-[3-[[(2,3-Dihydro-7-hydroxy-1,4-benzodioxin-2-yl)-methyl]-amino]-propoxy]-2H-1-benzopyran-2-on;
(S)-7-[3-[[(2,3-Dihydro-7-methoxy-1,4-benzodioxin-2-yl)-methyl]-amino]-propoxy]-2H-1-benzopyran-2-on;
(S)-7-[3-[[(2,3-Dihydro-6,7-methylendioxy-1,4-benzodioxin-2-yl)-methyl]-ethylamino]-propoxy]-2H-1-benzopyran-2-on;
(S)-7-[3-[[(2,3-Dihydro-6,7-methylendioxy-1,4-benzodioxin-2-yl)-methyl]-amino]-propoxy]-2H-1-benzopyran-2-on;
(S)-7-[3-[[(2,3-Dihydro-6-hydroxy-1,4-benzodioxin-2-yl)-methyl]-amino]-propoxy]-2H-1-benzopyran-2-on;
und ihren pharmazeutisch annehmbaren Salzen.

**10.** Verfahren nach Anspruch 1, welches durchgeführt wird zur Herstellung einer Verbindung, ausgewählt aus:
5-[3-[[2,3-Dihydro-1,4-benzodioxin-2-yl)-methyl]-amino]-propoxy]-2(1H)-chinolin;
(R)-7-[3-[[(2,3-Dihydro-6-hydroxy-1,4-benzodioxin-2-yl)-methyl]-amino]-propoxy]-2H-1-benzopyran-2-on;
und ihren pharmazeutisch annehmbaren Salzen.

**11.** Verfahren zur Herstellung einer pharmazeutischen Zubereitung, gekennzeichnet durch das Kombinieren oder Vereinigen einer Verbindung der Formel:

$Y^1$-$CH_2$-$NR^3$-$CH_2$-$Y^2$ ,

worin $Y^1$, $R^3$ und $Y^2$ wie in Anspruch 1 definiert sind, oder eines pharmazeutisch annehmbaren Salzes hievon mit einem pharmazeutischen Träger umfaßt.

**12.** Verwendung einer Verbindung der Formel:

$Y^1$-$CH_2$-$NR^3$-$CH_2$-$Y^2$ ,

worin $Y^1$, $R^3$ und $Y^2$ wie in Anspruch 1 definiert sind, oder eines pharmazeutisch annehmbaren Salzes hievon bei der Herstellung eines Medikaments zur Verwendung als Dopamin-Autorezeptor-Agonist.

**Revendications**

**Revendications pour les Etats contractants suivants : AT, BE, CH, DE, DK, FR, GB, IT, LI, LU, NL, PT, SE**

**1.** Composé de formule :

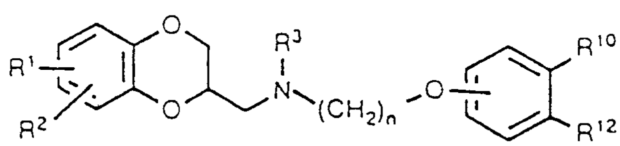

où :

$R^1$ et $R^2$ sont, indépendamment, hydrogène, alcoyle en 1 à 6 atomes de carbone, alcoxy en 1 à 6 atomes de carbone, aralcoxy en 7 à 12 atomes de carbone, alcanoyloxy en 2 à 6 atomes de carbone, hydroxyle, halo, amino, mono- ou dialcoylamino dans lequel chaque radical alcoyle contient de 1 à 6 atomes de carbone, alcanamido en 2 à 6 atomes de carbone ou sulfonamido, ou $R^1$ et $R^2$ forment ensemble méthylènedioxy, éthylènedioxy ou propylènedioxy;

$R^3$ est hydrogène ou alcoyle en 1 à 6 atomes de carbone;

n est un entier parmi 2, 3 ou 4;

$R^{12}$ est $NR^4R^5$ où $R^4$ et $R^5$ sont, indépendamment, hydrogène, alcoyle en 1 à 6 atomes de carbone, cycloalcoyle en 4 à 7 atomes de carbone, alcanoyle en 2 à 6 atomes de carbone, aroyle en 7 à 12 atomes de carbone, alcoylsulfonyle en 1 à 6 atomes de carbone ou arylsulfonyle en 6 à 10 atomes de carbone, ou $R^4$ et $R^5$ forment ensemble un cycle polyméthylène de 3 à 7 membres;

$R^{10}$ est hydrogène ou, lorsqu'il est pris avec $R^{12}$, $R^{10}$ et $R^{12}$ complètent le cycle à 6 membres :

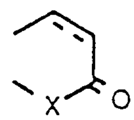

dans lequel X est O ou $NR^8$, dans lequel $R^8$ est hydrogène ou alcoyle en 1 à 6 atomes de carbone, ou un sel pharmaceutiquement acceptable de celui-ci.

**2.** Composé suivant la revendication 1, dans lequel $R^1$ et $R^2$ sont, indépendamment, hydrogène, fluoro, hydroxyle, alcoxy en 1 à 6 atomes de carbone ou alcanoyloxy en 2 à 6 atomes de carbone, ou forment ensemble un cycle méthylènedioxy, éthylènedioxy ou propylènedioxy; $R^4$ et $R^5$ sont, indépendamment, hydrogène ou alcoyle en 1 à 6 atomes de carbone, et le lien entre l'oxygène et la partie anilino est en position méta ou le lien $(CH_2)_n$-O est en position 7 du noyau coumarine ou 2-hydroxyquinoléine, ou un sel pharmaceutiquement acceptable de celui-ci.

**3.** Composé suivant la revendication 1, de formule :

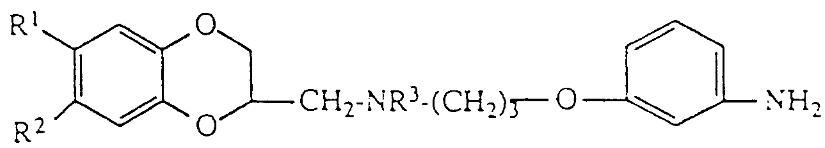

dans lequel :

$R^1$ et $R^2$ sont, indépendamment, hydrogène, fluoro, hydroxyle, alcoxy en 1 à 4 atomes de carbone, alcanoyloxy en 2 à 4 atomes de carbone ou forment ensemble un cycle alcoylènedioxy en 1 à 3 atomes de carbone;

$R^3$ est hydrogène ou alcoyle en 1 à 4 atomes de carbone, ou

un sel pharmaceutiquement acceptable de celui-ci, où la partie benzodioxanneméthanamine a la configuration S.

**4.** Composé suivant la revendication 1, de formule :

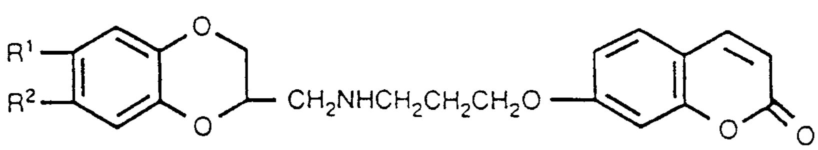

dans lequel $R^1$ et $R^2$ sont, indépendamment, hydrogène, fluoro, hydroxyle, alcoxy en 1 à 6 atomes de carbone, alcanoyloxy en 2 à 6 atomes de carbone, ou, pris ensemble, $R^1$ et $R^2$ sont méthylènedioxy, éthylènedioxy ou propylènedioxy, ou un sel pharmaceutiquement acceptable de celui-ci, dans lequel la partie benzodioxanneméthanamine a la configuration S.

**5.** Composé suivant la revendication 1, qui est choisi parmi :

la N-[3-(3-aminophénoxy)propyl]-2,3-dihydro-7-hydroxy-8-méthyl-1,4-benzodioxine-2-méthanamine;
la N-[3-(3-aminophénoxy)propyl]-2,3-dihydro-6,8-diméthoxy-1,4-benzodioxine-2-méthanamine;
la N-[3-(3-aminophénoxy)propyl]-2,3-dihydro-7-fluoro-1,4-benzodioxine-2-méthanamine;
la N-[3-(3-aminophénoxy)propyl]-2,3-dihydro-5,6-diméthoxy-1,4-benzodioxine-2-méthanamine;
la N-[3-(3-aminophénoxy)propyl]-2,3-dihydro-6,7-éthylènedioxy-1,4-benzodioxine-2-méthanamine;
la N-[3-(3-aminophénoxy)propyl]-2,3-dihydro-6,7-diméthoxy-1,4-benzodioxine-2-méthanamine;
la N-[3-(3-aminophénoxy)propyl]-2,3-dihydro-7-chloro-1,4-benzodioxine-2-méthanamine;
la N-[3-(3-aminophénoxy)propyl]-2,3-dihydro-8-hydroxy-1,4-benzodioxine-2-méthanamine;
la N-[3-(3-aminophénoxy)propyl]-2,3-dihydro-8-méthoxy-1,4-benzodioxine-2-méthanamine;

la N-[3-(3-aminophénoxy)propyl]-2,3-dihydro-6,7-méthylènedioxy-1,4-benzodioxine-2-méthanamine;
la N-[3-(3-aminophénoxy)propyl]-2,3-dihydro-7-méthoxy-1,4-benzodioxine-2-méthanamine;
la N-[3-(3-aminophénoxy)propyl]-2,3-dihydro-6-méthyl-7-méthoxy-1,4-benzodioxine-2-méthanamine;
la N-[3-(3-aminophénoxy)propyl]-2,3-dihydro-5-hydroxy-1,4-benzodioxine-2-méthanamine;
la N-[3-(3-aminophénoxy)propyl]-2,3-dihydro-6-hydroxy-1,4-benzodioxine-2-méthanamine;
la N-[3-(3-aminophénoxy)propyl]-2,3-dihydro-7-hydroxy-1,4-benzodioxine-2-méthanamine;
la N-[3-(3-aminophénoxy)propyl]-2,3-dihydro-6-méthyl-7-hydroxy-1,4-benzodioxine-2-méthanamine;
la N-[3-(3-aminophénoxy)propyl]-2,3-dihydro-5-méthoxy-1,4-benzodioxine-2-méthanamine, et
les sels pharmaceutiquement acceptables de celles-ci.

**6.** Composé suivant la revendication 1, qui est choisi parmi :
la (R)-N-[3-(3-aminophénoxy)propyl]-2,3-dihydro-7-hydroxy-1,4-benzodioxine-2-méthanamine;
la (R)-N-[3-(3-aminophénoxy)propyl]-2,3-dihydro-6,7-méthylènedioxy-1,4-benzodioxine-2-méthanamine;
la (R)-N-[3-(3-aminophénoxy)propyl]-2,3-dihydro-7-fluoro-1,4-benzodioxine-2-méthanamine;
la (R)-N-[3-(3-aminophénoxy)propyl]-2,3-dihydro-7-méthoxy-1,4-benzodioxine-2-méthanamine, et
les sels pharmaceutiquement acceptables de celles-ci.

**7.** Composé suivant la revendication 1, qui est choisi parmi :
la (S)-N-[3-(3-aminophénoxy)propyl]-2,3-dihydro-7-hydroxy-1,4-benzodioxine-2-méthanamine;
la (S)-N-[3-(3-aminophénoxy)propyl]-2,3-dihydro-6,7-méthylènedioxy-1,4-benzodioxine-2-méthanamine;
la (S)-N-[3-(3-aminophénoxy)propyl]-2,3-dihydro-7-fluoro-1,4-benzodioxine-2-méthanamine;
la (S)-N-[3-(3-aminophénoxy)propyl]-2,3-dihydro-7-méthoxy-1,4-benzodioxine-2-méthanamine, et
les sels pharmaceutiquement acceptables de celles-ci.

**8.** Composé suivant la revendication 1, qui est choisi parmi :
la 7-[3-[[(2,3-dihydro-1,4-benzodioxine-2-yl)méthyl]amino]propoxy]-2H-1-benzopyranne-2-one;
la 7-[3-[[(2,3-dihydro-6,8-méthoxy-1,4-benzodioxine-2-yl)méthyl]amino]propoxy]-2H-1-benzopyranne-2-one;
la 7-[3-[[(2,3-dihydro-5-méthoxy-1,4-benzodioxine-2-yl)méthyl]amino]propoxy]-2H-1-benzopyranne-2-one;
la 7-[3-[[(2,3-dihydro-7-chloro-1,4-benzodioxine-2-yl)méthyl]amino]propoxy]-2H-1-benzopyranne-2-one;
la 7-[3-[[(2,3-dihydro-7-phénylméthoxy-1,4-benzodioxine-2-yl)méthyl]amino]propoxy]-2H-1-benzopyranne-2-one;
la 7-[3-[[(2,3-dihydro-6-phénylméthoxy-1,4-benzodioxine-2-yl)méthyl]amino]propoxy]-2H-1-benzopyranne-2-one;
la 7-[3-[[(2,3-dihydro-6-hydroxy-1,4-benzodioxine-2-yl)méthyl]amino]propoxy]-2H-1-benzopyranne-2-one;
la 7-[3-[[(2,3-dihydro-7-hydroxy-1,4-benzodioxine-2-yl)méthyl]amino]propoxy]-2H-1-benzopyranne-2-one;
la 7-[3-[[(2,3-dihydro-7-méthoxy-1,4-benzodioxine-2-yl)méthyl]amino]propoxy]-2H-1-benzopyranne-2-one;
la 7-[3-[[(2,3-dihydro-6-acétoxy-1,4-benzodioxine-2-yl)méthyl]amino]propoxy]-2H-1-benzopyranne-2-one;
la 7-[3-[[(2,3-dihydro-7-hydroxy-1,4-benzodioxine-2-yl)méthyl]éthylamino]propoxy-2H-1-benzopyranne-2-one;
la 7-[3-[[(2,3-dihydro-1,4-benzodioxine-2-yl)méthyl]éthylamino]propoxy]-2H-1-benzopyranne-2-one;
la 7-[3-[[(2,3-dihydro-7-méthoxy-1,4-benzodioxine-2-yl)méthyl]méthylamino]propoxy]-2H-1-benzopyranne-2-one;
la 7-[3-[[(2,3-dihydro-6-hydroxy-1,4-benzodioxine-2-yl)méthyl]méthylamino]propoxy]-2H-1-benzopyranne-2-one, et
les sels pharmaceutiquement acceptables de celles-ci.

**9.** Composé suivant la revendication 1, qui est choisi parmi :
la (S)-7-[3-[[(2,3-dihydro-7-hydroxy-1,4-benzodioxine-2-yl)méthyl]éthylamino]propoxy]-2H-1-benzopyranne-2-one;

la (S)-7-[3-[[(2,3-dihydro-7-hydroxy-1,4-benzodioxine-2-yl)méthyl]amino]propoxy]-2H-1-benzopyranne-2-one;

la (S)-7-[3-[[(2,3-dihydro-7-méthoxy-1,4-benzodioxine-2-yl)méthyl]amino]propoxy]-2H-1-benzopyranne-2-one;

la (S)-7-[3-[[(2,3-dihydro-6,7-méthylènedioxy-1,4-benzodloxine-2-yl)méthyl]éthylamino]propoxy]-2H-1-benzopyranne-2-one;

la (S)-7-[3-[[(2,3-dihydro-6,7-méthylènedioxy-1,4-benzodioxine-2-yl)méthyl]amino]propoxy]-2H-1-benzopyranne-2-one;

la (S)-7-[3-[[(2,3-dihydro-6-hydroxy-1,4-benzodioxine-2-yl)méthyl]amino]propoxy]-2H-1-benzopyranne-2-one, et

leurs sels pharmaceutiquement acceptables.

**10.** Composé suivant la revendication 1, qui est choisi parmi :

la 5-[3-[[(2,3-dihydro-1,4-benzodioxine-2-yl)méthyl]amino]propoxy]-2(1H)-quinoléine;

la (R)-7-[3-[[(2,3-dihydro-6-hydroxy-1,4-benzodioxine-2-yl)méthyl]amino]propoxy]-2H-1-benzopyranne-2-one, et

leurs sels pharmaceutiquement acceptables.

**11.** Composition pharmaceutique comprenant un composé suivant l'une quelconque des revendications 1 à 10, en combinaison ou en association avec un support pharmaceutique.

**Revendications pour les Etats contractants suivants : ES, GR**

**1.** Procédé de préparation d'un composé de formule :

$Y^1\text{-}CH_2\text{-}NR^3\text{-}CH_2\text{-}Y^2$

ou d'un sel pharmaceutiquement acceptable de celui-ci, où $R^3$ est hydrogène ou alcoyle en 1 à 6 atomes de carbone; $Y^1$ et $Y^2$ sont un radical ayant la formule :

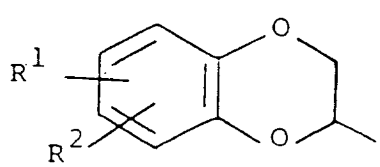

où :

$R^1$ et $R^2$ sont, indépendamment, hydrogène, alcoyle en 1 à 6 atomes de carbone, alcoxy en 1 à 6 atomes de carbone, aralcoxy en 7 à 12 atomes de carbone, alcanoyloxy en 2 à 5 atomes de carbone, hydroxyle, halo, amino, mono- ou dialcoylamino où chaque radical alcoyle contient de 1 à 6 atomes de carbone, alcanamido en 2 à 6 atomes de carbone, sulfonamido, ou $R^1$ et $R^2$ forment ensemble un méthylènedioxy, éthylènedioxy ou propylènedioxy, et

l'autre des $Y^1$ et $Y^2$ est un radical ayant la formule :

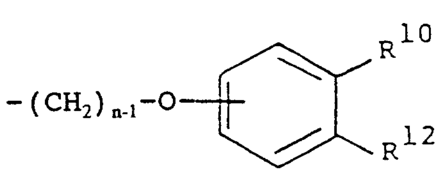

où :

n est l'un des entiers 2, 3 ou 4;

$R^{12}$ est $NR^4R^5$ dans lequel $R^4$ et $R^5$ sont, indépendamment, hydrogène, alcoyle en 1 à 6 atomes de carbone, cycloalcoyle en 4 à 7 atomes de carbone, alcanoyle en 2 à 6 atomes de carbone, aroyle en 7 à 12 atomes de carbone, alcoylsulfonyle en 1 à 6 atomes de carbone ou arylsulfonyle en 6 à 10

atomes de carbone, ou $R^4$ et $R^5$ forment ensemble un cycle polyméthylène de 3 à 7 membres;

$R^{10}$ est hydrogène ou, pris avec $R^{12}$, $R^{10}$ et $R^{12}$ complètent le cycle à 6 membres :

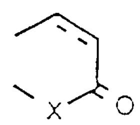

dans lequel la ligne en traits interrompus signifie une insaturation facultative et X est O ou $NR^8$, où $R^8$ est hydrogène ou alcoyle en 1 à 6 atomes de carbone; dans lequel :

(a) une amine ayant la formule :

$Y^1-CH_2-NHR^3$

est N-alcoylée pour introduire un radical alcoyle substitué ayant la formule $-CH_2-Y^2$ où $Y^1$, $Y^2$ et $R^3$ sont tels que définis ci-dessus, sauf qu'un radical amino ou monoalcoylamino représenté par $R^1$, $R^2$ ou $-NR^4R^5$ ou un radical hydroxyle représenté par $R^1$ ou $R^2$ peut être temporairement protégé par un groupe protecteur pouvant être éliminé, ou

(b) une amine ayant la formule :

$Y^1-CH_2-NHR^3$

est N-alcoylée de manière réductrice au moyen d'un aldéhyde ayant la formule $Y^2-CHO$, où $Y^1$, $Y^2$ et $R^3$ sont tels que définis ci-dessus, ou

(c) une amine ayant la formule :

$Y^1-CO-NR^3-CH_2-Y^2$

où :

$Y^1$, $Y^2$ et $R^3$ sont tels que définis ci-dessus, est réduite pour convertir le radical carbonyle de celle-ci en méthylène, ou

(d) un composé nitro ayant la formule :

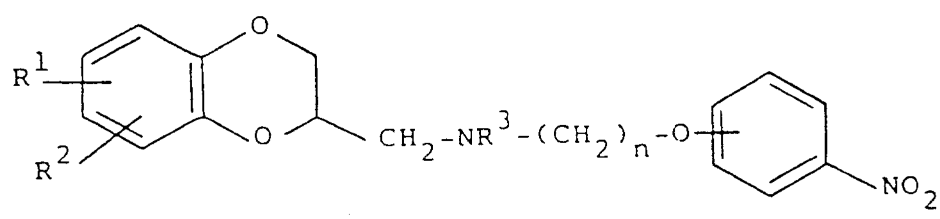

où :

$R^1$, $R^2$, $R^3$ et n sont tels que définis ci-dessus, est réduit pour convertir le radical nitro en amino, ou

(e) une amine ayant la formule :

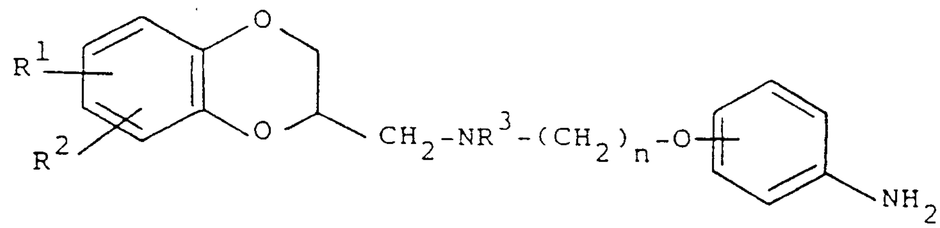

est soumise à une alcoylation, une acylation ou une alcoyl- ou arylsulfonylation pour convertir le $-NH_2$ en une autre signification de $-NR^4R^5$, et, si approprié, un groupe protecteur est éliminé et, si désiré, un composé obtenu est converti en un sel pharmaceutiquement acceptable de celui-ci ou, si désiré, un sel est converti en une base libre.

**2.** Procédé suivant la revendication 1, dans lequel $R^1$ et $R^2$ sont, indépendamment, hydrogène, fluoro, hydroxyle, alcoxy en 1 à 6 atomes de carbone ou alcanoyloxy en 2 à 6 atomes de carbone, ou forment ensemble un cycle méthylènedioxy, éthylènedioxy ou propylènedioxy; $R^4$ et $R^5$ sont, indépendamment, hydrogène ou alcoyle en 1 à 6 atomes de carbone, et le lien entre l'oxygène et la partie anilino est en position méta ou le lien $(CH_2)_n$-O est en position 7 du noyau coumarine ou 2-hydroxyquinoléine.

**3.** Procédé suivant la revendication 1, réalisé pour préparé un composé de formule :

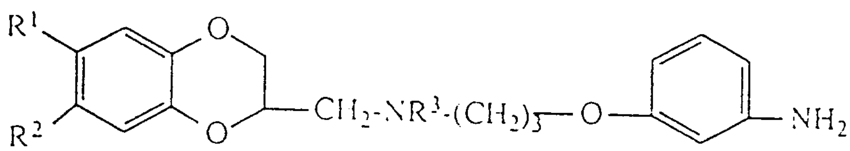

dans lequel :

$R^1$ et $R^2$ sont, indépendamment, hydrogène, fluoro, hydroxyle, alcoxy en 1 à 4 atomes de carbone, alcanoyloxy en 2 à 4 atomes de carbone ou forment ensemble un cycle alcoylènedioxy de 1 à 3 atomes de carbone;

$R^3$ et hydrogène ou alcoyle en 1 à 4 atomes de carbone, ou

un sel pharmaceutiquement acceptable de celui-ci, où la partie benzodioxanneméthanamine a la configuration S.

**4.** Procédé suivant la revendication 1, réalisé pour préparer un composé de formule :

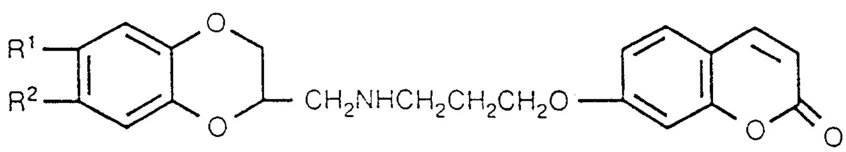

dans lequel $R^1$ et $R^2$ sont, indépendamment, hydrogène, fluoro, hydroxyle, alcoxy en 1 à 6 atomes de carbone, alcanoyloxy en 2 à 6 atomes de carbone, ou, pris ensemble, $R^1$ et $R^2$ sont méthylènedioxy, éthylènedioxy ou propylènedioxy, ou un sel pharmaceutiquement acceptable de celui-ci, dans lequel la partie benzodioxanneméthanamine a la configuration S.

**5.** Procédé suivant la revendication 1, réalisé pour préparer un composé choisi parmi :

la N-[3-(3-aminophénoxy)propyl]-2,3-dihydro-7-hydroxy-8-méthyl-1,4-benzodioxine-2-méthanamine;
la N-[3-(3-aminophénoxy)propyl]-2,3-dihydro-6,8-diméthoxy-1,4-benzodioxine-2-méthanamine;
la N-[3-(3-aminophénoxy)propyl]-2,3-dihydro-7-fluoro-1,4-benzodioxine-2-méthanamine;
la N-[3-(3-aminophénoxy)propyl]-2,3-dihydro-5,6-diméthoxy-1,4-benzodioxine-2-méthanamine;
la N-[3-(3-aminophénoxy)propyl]-2,3-dihydro-6,7-éthylènedioxy-1,4-benzodioxine-2-méthanamine;
la N-[3-(3-aminophénoxy)propyl]-2,3-dihydro-6,7-diméthoxy-1,4-benzodioxine-2-méthanamine;
la N-[3-(3-aminophénoxy)propyl]-2,3-dihydro-7-chloro-1,4-benzodioxine-2-méthanamine;
la N-[3-(3-aminophénoxy)propyl]-2,3-dihydro-8-hydroxy-1,4-benzodioxine-2-méthanamine;
la N-[3-(3-aminophénoxy)propyl]-2,3-dihydro-8-méthoxy-1,4-benzodioxine-2-méthanamine;
la N-[3-(3-aminophénoxy)propyl]-2,3-dihydro-6,7-méthylènedioxy-1,4-benzodioxine-2-méthanamine;
la N-[3-(3-aminophénoxy)propyl]-2,3-dihydro-7-méthoxy-1,4-benzodioxine-2-méthanamine;
la N-[3-(3-aminophénoxy)propyl]-2,3-dihydro-6-méthyl-7-méthoxy-1,4-benzodioxine-2-méthanamine;
la N-[3-(3-aminophénoxy)propyl]-2,3-dihydro-5-hydroxy-1,4-benzodioxine-2-méthanamine;

la N-[3-(3-aminophénoxy)propyl]-2,3-dihydro-6-hydroxy-1,4-benzodioxine-2-méthanamine;
la N-[3-(3-aminophénoxy)propyl]-2,3-dihydro-7-hydroxy-1,4-benzodioxine-2-méthanamine;
la N-[3-(3-aminophénoxy)propyl]-2,3-dihydro-6-méthyl-7-hydroxy-1,4-benzodioxine-2-méthanamine;
la N-[3-(3-aminophénoxy)propyl]-2,3-dihydro-5-méthoxy-1,4-benzodioxine-2-méthanamine, et
les sels pharmaceutiquement acceptables de celles-ci.

6. Procédé suivant la revendication 1, réalisé pour préparer un composé choisi parmi :
la (R)-N-[3-(3-aminophénoxy)propyl]-2,3-dihydro-7-hydroxy-1,4-benzodioxine-2-méthanamine;
la (R)-N-[3-(3-aminophénoxy)propyl]-2,3-dihydro-6,7-méthylènedioxy-1,4-benzodioxine-2-méthanamine;
la (R)-N-[3-(3-aminophénoxy)propyl]-2,3-dihydro-7-fluoro-1,4-benzodioxine-2-méthanamine;
la (R)-N-[3-(3-aminophénoxy)propyl]-2,3-dihydro-7-méthoxy-1,4-benzodioxine-2-méthanamine, et
les sels pharmaceutiquement acceptables de celles-ci.

7. Procédé suivant la revendication 1, réalisé pour préparer un composé choisi parmi :
la (S)-N-[3-(3-aminophénoxy)propyl]-2,3-dihydro-7-hydroxy-1,4-benzodioxine-2-méthanamine;
la (S)-N-[3-(3-aminophénoxy)propyl]-2,3-dihydro-6,7-méthylènedioxy-1,4-benzodioxine-2-méthanamine;
la (S)-N-[3-(3-aminophénoxy)propyl]-2,3-dihydro-7-fluoro-1,4-benzodioxine-2-méthanamine;
la (S)-N-[3-(3-aminophénoxy)propyl]-2,3-dihydro-7-méthoxy-1,4-benzodioxine-2-méthanamine, et
les sels pharmaceutiquement acceptables de celles-ci.

8. Procédé suivant la revendication 1, réalisé pour préparer un composé choisi parmi :
la 7-[3-[[(2,3-dihydro-1,4-benzodioxine-2-yl)méthyl]amino]propoxy]-2H-1-benzopyranne-2-one;
la 7-[3-[[(2,3-dihydro-6,8-méthoxy-1,4-benzodioxine-2-yl)méthyl]amino]propoxy]-2H-1-benzopyranne-2-one;
la 7-[3-[[(2,3-dihydro-5-méthoxy-1,4-benzodioxine-2-yl)méthyl]amino]propoxy]-2H-1-benzopyranne-2-one;
la 7-[3-[[(2,3-dihydro-7-chloro-1,4-benzodioxine-2-yl)méthyl]amino]propoxy]-2H-1-benzopyranne-2-one;
la 7-[3-[[(2,3-dihydro-7-phénylméthoxy-1,4-benzodioxine-2-yl)méthyl]amino]propoxy]-2H-1-benzopyranne-2-one;
la 7-[3-[[(2,3-dihydro-6-phénylméthoxy-1,4-benzodioxine-2-yl)méthyl]amino]propoxy]-2H-1-benzopyranne-2-one;
la 7-[3-[[(2,3-dihydro-6-hydroxy-1,4-benzodioxine-2-yl)méthyl]amino]propoxy]-2H-1-benzopyranne-2-one;
la 7-[3-[[(2,3-dihydro-7-hydroxy-1,4-benzodioxine-2-yl)méthyl]amino]propoxy]-2H-1-benzopyranne-2-one;
la 7-[3-[[(2,3-dihydro-7-méthoxy-1,4-benzodioxine-2-yl)méthyl]amino]propoxy]-2H-1-benzopyranne-2-one;
la 7-[3-[[(2,3-dihydro-6-acétoxy-1,4-benzodioxine-2-yl)méthyl]amino]propoxy]-2H-1-benzopyranne-2-one;
la 7-[3-[[(2,3-dihydro-7-hydroxy-1,4-benzodioxine-2-yl)méthyl]éthylamino]propoxy-2H-1-benzopyranne-2-one;
la 7-[3-[[(2,3-dihydro-1,4-benzodioxine-2-yl)méthyl]éthylamino]propoxy]-2H-1-benzopyranne-2-one;
la 7-[3-[[(2,3-dihydro-7-méthoxy-1,4-benzodioxine-2-yl)méthyl]méthylamino]propoxy]-2H-1-benzopyranne-2-one;
la 7-[3-[[(2,3-dihydro-6-hydroxy-1,4-benzodioxine-2-yl)méthyl]méthylamino]propoxy]-2H-1-benzopyranne-2-one, et
les sels pharmaceutiquement acceptables de celles-ci.

9. Procédé suivant la revendication 1, réalisé pour préparer un composé choisi parmi :
la (S)-7-[3-[[(2,3-dihydro-7-hydroxy-1,4-benzodioxine-2-yl)méthyl]éthylamino]propoxy]-2H-1-benzopyranne-2-one;
la (S)-7-[3-[[(2,3-dihydro-7-hydroxy-1,4-benzodioxine-2-yl)méthyl]amino]propoxy]-2H-1-benzopyranne-2-one;
la (S)-7-[3-[[(2,3-dihydro-7-méthoxy-1,4-benzodioxine-2-yl)méthyl]amino]propoxy]-2H-1-benzopyranne-2-one;

la (S)-7-[3-[[(2,3-dihydro-6,7-méthylènedioxy-1,4-benzodioxine-2-yl)méthyl]éthylamino]propoxy]-2H-1-benzopyranne-2-one;

la (S)-7-[3-[[(2,3-dihydro-6,7-méthylènedioxy-1,4-benzodioxine-2-yl)méthyl]amino]propoxy]-2H-1-benzopyranne-2-one;

la (S)-7-[3-[[(2,3-dihydro-6-hydroxy-1,4-benzodioxine-2-yl)méthyl]amino]propoxy]-2H-1-benzopyranne-2-one, et

leurs sels pharmaceutiquement acceptables.

**10.** Procédé suivant la revendication 1, réalisé pour préparer un composé choisi parmi :

la 5-[3-[[(2,3-dihydro-1,4-benzodioxine-2-yl)méthyl]amino]propoxy]-2(1H)-quinoléine;

la (R)-7-[3-[[(2,3-dihydro-6-hydroxy-1,4-benzodioxine-2-yl)méthyl]amino]propoxy]-2H-1-benzopyranne-2-one, et

leurs sels pharmaceutiquement acceptables.

**11.** Procédé de préparation d'une composition pharmaceutique, caractérisé par la mise en commun ou l'association d'un composé ayant la formule :

$Y^1\text{-}CH_2\text{-}NR^3\text{-}CH_2\text{-}Y^2$

où :

$Y^1$, $R^3$ et $Y^2$ sont tels que définis à la revendication 1, ou d'un sel pharmaceutiquement acceptable de celui-ci, avec un support pharmaceutique.

**12.** Utilisation d'un composé ayant la formule :

$Y^1\text{-}CH_2\text{-}NR^3\text{-}CH_2\text{-}Y^2$

où :

$Y^1$, $R^3$ et $Y^2$ sont tels que définis à la revendication 1, ou d'un sel pharmaceutiquement acceptable de celui-ci, pour préparer un médicament à utiliser comme agoniste de l'autorécepteur de la dopamine.